# EUROPEAN PATENT SPECIFICATION

(11) **EP 2 800 758 B1**
(45) Date of publication and mention of the grant of the patent: **01.08.2018**
(21) Application number: 13700185.5
(22) Date of filing: 03.01.2013
(51) Int. Cl.: C07K 5/087

(54) **N-ACYLPEPTIDE DERIVATIVES AND THEIR USES**
N-ACYLPEPTIDDERIVATE UND DEREN VERWENDUNG
DÉRIVÉS N-ACYLPEPTIDIQUES ET LEURS UTILISATIONS

(30) Priority: 03.01.2012 US 201261582675 P
(43) Date of publication of application: 12.11.2014
(73) Proprietor: NeoStrata Company, Inc., Princeton, NJ 08540 (US)
(72) Inventor: YU, Ruey, J., Chalfont, PA 18914 (US); VAN SCOTT, Eugene, J., Abington, PA 19001 (US)
(74) Representative: Grünecker Patent- und Rechtsanwälte PartG mbB
(86) International application number: PCT/US2013/020015
(87) International publication number: WO 2013/103634

(56) References cited:
- WO-A1-2009/148947
- WO-A1-2009/148947
- WO-A1-2010/065342
- WO-A1-2010/065342
- US-A1- 2003 194 445
- US-A1- 2003 194 445
- US-A1- 2011 206 752
- US-B1- 6 620 419
- KUI-THONG TAN ET AL: "Design, Synthesis, and Characterization of Peptide-Based Rab Geranylgeranyl Transferase Inhibitors", JOURNAL OF MEDICINAL CHEMISTRY, vol. 52, no. 24, 24 December 2009 (2009-12-24), pages 8025-8037, XP055032644, ISSN: 0022-2623, DOI: 10.1021/jm901117d -& KUI-THONG TAN ET AL: "Supporting Info for: Design, Synthesis, and Characterization of Peptide-Based Rab Geranylgeranyl Transferase Inhibitors", JOURNAL OF MEDICINAL CHEMISTRY, vol. 52, no. 24, 24 December 2009 (2009-12-24), pages 8025-8037, XP055256432, US ISSN: 0022-2623, DOI: 10.1021/jm901117d
- PHILIPP KRATTIGER ET AL: "Water-Soluble Diketopiperazine Receptors - Selective Recognition of Arginine-Rich Peptides", SYNLETT, no. 4, 1 January 2005 (2005-01-01), pages 706-708, XP055255727, DE ISSN: 0936-5214, DOI: 10.1055/s-2005-862395
- M. L. HUANG ET AL: "Biomimetic peptoid oligomers as dual-action antifreeze agents", PROCEEDINGS OF THE NATIONAL ACADEMY OF SCIENCES, vol. 109, no. 49, 19 November 2012 (2012-11-19), pages 19922-19927, XP055225052, US ISSN: 0027-8424, DOI: 10.1073/pnas.1212826109
- RINGSEIS R ET AL: "Tripeptides from dietary proteins inhibit TNFalpha-induced monocyte adhesion to human aortic endothelial cells", REGULATORY PEPTIDES, ELSEVIER SCIENCE BV, NL, vol. 154, no. 1-3, 10 April 2009 (2009-04-10), pages 91-96, XP026079178, ISSN: 0167-0115, DOI: 10.1016/J.REGPEP.2008.10.009 [retrieved on 2008-11-08]
- Ernawati Sinaga ET AL: "Increasing Paracellular Porosity by E-Cadherin Peptides: Discovery of Bulge and Groove Regions in the EC1-Domain of E-Cadherin", Pharmaceutical Research, 1 August 2002 (2002-08-01), pages 1170-1179, XP055256024, New York DOI: 10.1023/A:1019850226631 Retrieved from the Internet: URL:http://rd.springer.com/article/10.1023 /A:1019850226631 [retrieved on 2016-03-08]
- P L CHEN ET AL: "[Synthesis and biological activity of some Val(Ala)-Tyr and Val-Tyr-Tyr peptides]", YAO XUE XUE BAO = ACTA PHARMACEUTICA SINICA, vol. 27, no. 12, 1 January 1992 (1992-01-01), pages 895-902, XP055256035,
- LIVANT D L ET AL: "ANTI-INVASIVE, ANTITUMORIGENIC, AND ANTIMETASTATIC ACTIVITIES OF THE PHSCN SEQUENCE IN PROSTATE CARCINOMA", CANCER RESEARCH, AMERICAN ASSOCIATION FOR CANCER RESEARCH, US, vol. 60, no. 2, 15 January 2000 (2000-01-15), pages 309-320, XP001147065, ISSN: 0008-5472
- DAVID G. LAMBERT: "The nociceptin/orphanin FQ receptor: a target with broad therapeutic potential", NATURE REVIEWS DRUG DISCOVERY, vol. 7, no. 8, 1 August 2008 (2008-08-01), pages 694-710, XP055069773, ISSN: 1474-1776, DOI: 10.1038/nrd2572
- T. E. Gottschalck & G. N. McEwen: "International Cosmetic Ingredient Dictionary and Handbook", 2006, The Cosmetic, Toiletry, and Fragrance Association, Washington, XP009170884, ISBN: 1-882621-36-0 pages 2712-2712, page 2712, all acetyl peptides, myristoyl peptides and palmitoyl peptides
- CLEMENS SORG ET AL: "Stufenweise Synthese von Oligotyrosinpeptiden", LIEBIGS ANN. CHEM., vol. 734, 1 January 1970 (1970-01-01), pages 180-186, XP055069786,
- SANDHU ET AL: "Peptide binding specificity of the chaperone calreticulin", BIOCHIMICA ET BIOPHYSICA ACTA (BBA) - PROTEINS & PROTEOMICS, ELSEVIER, NETHERLANDS, vol. 1774, no. 6, 5 June 2007 (2007-06-05) , pages 701-713, XP022104837, ISSN: 1570-9639, DOI: 10.1016/J.BBAPAP.2007.03.019
- MICHAEL FITZEN ET AL: "Peptide-binding specificity of the prosurfactant protein C Brichos domain analyzed by electrospray ionization mass spectrometry", RAPID COMMUNICATIONS IN MASS SPECTROMETRY, vol. 23, no. 22, 1 November 2009 (2009-11-01), pages 3591-3598, XP055077611, ISSN: 0951-4198, DOI: 10.1002/rcm.4282
- KATHARINA WOITHE ET AL: "Exploring the substrate specificity of OxyB, a phenol coupling P450 enzyme involved in vancomycin biosynthesis", ORGANIC & BIOMOLECULAR CHEMISTRY, vol. 6, no. 16, 1 August 2008 (2008-08-01) , pages 2861-2867, XP055077508, ISSN: 1477-0520, DOI: 10.1039/b805956j
- KUI-THONG TAN ET AL: "Design, Synthesis, and Characterization of Peptide-Based Rab Geranylgeranyl Transferase Inhibitors", JOURNAL OF MEDICINAL CHEMISTRY, vol. 52, no. 24, 24 December 2009 (2009-12-24), pages 8025-8037, XP055032644, ISSN: 0022-2623, DOI: 10.1021/jm901117d -& KUI-THONG TAN ET AL: "Supporting Info for: Design, Synthesis, and Characterization of Peptide-Based Rab Geranylgeranyl Transferase Inhibitors", JOURNAL OF MEDICINAL CHEMISTRY, vol. 52, no. 24, 24 December 2009 (2009-12-24), pages 8025-8037, XP055256432, US ISSN: 0022-2623, DOI: 10.1021/jm901117d
- PHILIPP KRATTIGER ET AL: "Water-Soluble Diketopiperazine Receptors - Selective Recognition of Arginine-Rich Peptides", SYNLETT, no. 4, 1 January 2005 (2005-01-01), pages 706-708, XP055255727, DE ISSN: 0936-5214, DOI: 10.1055/s-2005-862395
- M. L. HUANG ET AL: "Biomimetic peptoid oligomers as dual-action antifreeze agents", PROCEEDINGS OF THE NATIONAL ACADEMY OF SCIENCES, vol. 109, no. 49, 19 November 2012 (2012-11-19), pages 19922-19927, XP055225052, US ISSN: 0027-8424, DOI: 10.1073/pnas.1212826109
- RINGSEIS R ET AL: "Tripeptides from dietary proteins inhibit TNFalpha-induced monocyte adhesion to human aortic endothelial cells", REGULATORY PEPTIDES, ELSEVIER SCIENCE BV, NL, vol. 154, no. 1-3, 10 April 2009 (2009-04-10), pages 91-96, XP026079178, ISSN: 0167-0115, DOI: 10.1016/J.REGPEP.2008.10.009 [retrieved on 2008-11-08]
- Ernawati Sinaga ET AL: "Increasing Paracellular Porosity by E-Cadherin Peptides: Discovery of Bulge and Groove Regions in the EC1-Domain of E-Cadherin", Pharmaceutical Research, 1 August 2002 (2002-08-01), pages 1170-1179, XP055256024, New York DOI: 10.1023/A:1019850226631 Retrieved from the Internet: URL:http://rd.springer.com/article/10.1023 /A:1019850226631 [retrieved on 2016-03-08]
- P L CHEN ET AL: "[Synthesis and biological activity of some Val(Ala)-Tyr and Val-Tyr-Tyr peptides]", YAO XUE XUE BAO = ACTA PHARMACEUTICA SINICA, vol. 27, no. 12, 1 January 1992 (1992-01-01), pages 895-902, XP055256035,

## Description

### FIELD OF THE INVENTION

The embodiments described herein relate to novel compounds, compositions and uses of the compositions comprising N-acylpeptide derivatives for systemic or topical administration to a mammal to alleviate or improve diseases, symptoms or syndromes associated with tumors, cancers, immune, nervous, vascular, musculoskeletal, cutaneous system, or other tissues and systems.

### BACKGROUND OF THE INVENTION

In Handbook of Neurochemistry and Molecular Neurobiology 3rd Ed. "Amino Acids and Peptides in the Nervous System" by Oja et al. Springer Science 2007, page 401-411, Reichelt describes in "Low Molecular Weight Peptides" endogenous peptides. These peptides are Glu-Ala-Gly and Glu-Cys-Gly isolated from monkey brain; Glu-Met-Cys-Gly (SEQ ID NO:1) isolated from ox brain; N-acetyl (N-Ac) or N-pyroglutamyl (N-PyroE) peptides, such as N-Ac-Asp-Gly-Ser, N-Ac-Asp-Glu-Gly, N-Ac-Asp-Glu-Asp, N-PyroE-His-Pro-NH₂, N-PyroE-His-Trp-Ser-Tyr-Gly-Leu-Arg-Pro-NH₂ (SEQ ID NO:2) are naturally occurring small peptides acting as signal molecules in the body. However, there is no description or report about the N-acylpeptide derivatives of the present invention.

In a publication by Tuszynski et al. Experimental and Molecular Pathology: 91, 608-613, 2011 "G-protein coupled receptor-associated sorting protein 1 (GASP-1), a potential biomarker in breast cancer", a two dimensional high performance liquid electrophoresis method (2D-HPLE, US 7,326,326) was used to identify serum biomarkers associated with different stages of breast cancer. Based on this technology, a specific fragment or peptide of GASP-1 was found in sera of patients with early stage of breast cancer, but absent in sera of normal subjects. One of the fragments or peptides was identified as a pentadecapeptide containing 15 amino acid residues with a free amino group and a free carboxyl group each at one end of the peptide, and confirmed as Glu-Ala-Ser-Pro-Glu-Ala-Val-Ala-Gly-Val-Gly-Phe-Glu-Ser-Lys (GASP-1 P15, SEQ ID NO:3). In Chang et al. U.S. Patent Application No. 13/384,014, published on 7/19/12, entitled "Serum Markers Associated with Early and Other Stages of Breast Cancer" it was described that in addition to the above pentadecapeptide, a hexadecapeptide containing 16 amino acid residues with a free amino group and a free carboxyl group at both ends of the peptide, and confirmed as Glu-Glu-Ala-Ser-Pro-Glu-Ala-Val-Ala-Gly-Val-Gly-Phe-Glu-Ser-Lys (GASP-1 P16, SEQ ID NO:4). It has been reported that GASP-1 is also highly expressed in the sera of patients having brain cancer, lung cancer, liver cancer, or triple negative breast cancer. However, there is no description or report about N-acylpeptide derivatives of the present invention.

In a publication by Zhou et al. J. Cellular Biochemistry 92:125-146, 2004; entitled "Cloning and Characterization of Angiocidin, a Tumor Cell Binding Protein for Thrombospondin-1", a hexapeptide, Cys-Ser-Val-Thr-Cys-Gly (SEQ ID NO:5), a sequence or part of thrombospodin-1 molecule was shown to function as a tumor cell adhesion domain, and also to bind angiocidin. The thrombospodin-1 is a matrix glycoprotein in the body, and has been implicated in mechanisms of tumor progression. However, there is no description or report about N-acylpeptide derivatives of the present invention.

In a publication by Sabherwal et al. Experimental Cell Research 312:2443-2453, 2006; entitled " Integrin α2β1 Mediates the Anti-Angiogenic and Anti-Tumor Activities of Angiocidin, a Novel Tumor-Associated Protein" an eicosapeptide, a peptide containing 20 amino acid residues, FCTGIRVAHLALKHRQGKNH (SEQ ID NO:6) is a sequence or part of angiocidin protein (from No. 87 to No. 106 in amino acid sequence), which has been found to mediate the anti-tumor activity of angiocidin. The angiocidin protein was initially isolated from lung carcinoma in 1993, and was later cloned based on the full-length cDNA in bacteria. This recombinant protein was referred to as angiocidin.

Gottschalck and McEwen, international cosmetic ingredient dictionary and handbook, 2006, the cosmetic, toiletry and fragrance association, Washington, page 2712 discloses an acetyl tripeptide for use in the treatment of disorders, diseases, symptoms or syndromes. Fitzen et al, RAPID COMMUN MASS SPECTROM, 23(22), 2009, 3591-3598 and Woithe et al, ORG BIOMOL CHEM, 6, 2008, 2861-2867 describe compositions comprising an N-acylpeptide derivative of the present invention. However, said compositions are neither intended nor suitable for topical or systemic administration.

### BRIEF SUMMARY OF THE INVENTION

It has been discovered in the present invention that novel N-acylpeptide derivatives and compositions comprising the N-acylpeptide derivatives are therapeutically effective for topical or systemic administration to alleviate or improve conditions, disorders, diseases, symptoms or syndromes associated with tumors, cancers, immune, nervous, vascular, musculoskeletal, cutaneous system, or other tissues or systems in a subject.

In one general aspect, the present invention relates a composition comprising to a peptide derivative having the following generic Formula (I):

R₁-AAB-(AAA)ₙ-AAC-R₂ Formula (I)

as defined in claim 1.

A typical acyl radical is acetyl (Ac), propanoyl (Pr), or benzoyl (Bz). A typical group attached to the carboxyl-terminal amino acid residue is OH, OEt, NH₂, NHOH, or NHNH₂.

One general aspect of the invention relates to a composition for topical or systemic administration to a mammal, which comprisese a pharmaceutically or cosmetically acceptable carrier and a therapeutically effective amount of a peptide derivative having the generic Formula (I) described above, the composition for use as of claims 3 and 6.

Other aspects, features and advantages of the invention will be apparent from the following disclosure, including the detailed description of the invention and its preferred embodiments, and the appended claims.

### DESCRIPTION OF THE INVENTION

Various publications, articles and patents are cited or described in the background and throughout the specification; each of these references is herein incorporated by reference in its entirety. Discussion of documents, acts, materials, devices, articles or the like which has been included in the present specification is for the purpose of providing context for the present invention. Such discussion is not an admission that any or all of these matters form part of the prior art with respect to any inventions disclosed or claimed.

Unless defined otherwise, all technical and scientific terms used herein have the same meaning as commonly understood by one of ordinary skill in the art to which this invention pertains. Otherwise, certain terms used herein have the meanings as set forth in the specification. All patents, published patent applications and publications cited herein are incorporated by reference as if set forth fully herein. It must be noted that as used herein and in the appended claims, the singular forms "a," "an," and "the" include plural reference unless the context clearly dictates otherwise.

Common or certain knowledge, scientific and medical terminologies can be readily found via internet, textbooks of chemistry, biochemistry, medicinal chemistry, pharmacology, dermatology and general medicine. The following are some examples. Robert K. Murray et al. eds. "Harper's Illustrated Biochemistry" 26th edn. Vol. I-II, McGraw Hill, 2003. Laurence L. Brunton et al. eds. "Goodman & Gilman's The Pharmacological Basis of Therapeutics" 12th edn. McGraw Hill Medical, 2011. Klaus Wolff et al. eds. "Fitzpatrick's Dermatology in General Medicine" 7th edn. Vol. I-II, McGraw Hill Medical, New York, 2008. Tony Burns et al. eds. "Rook's Textbook of Dermatology" 8th edn. Vol. I-IV, Wiley-Blackwell, 2010. Anthony S. Fauci et al. eds. "Harrison's Principles of Internal Medicine" 17th edn, McGraw Hill Medical, New York, 2008.

An amino acid is an organic acid having one or more than one alkaline radical such as amino, guanidino, imino, or hydrazine radical attached at any carbon atom other than carbon one. There are 20 common amino acids which are represented by chemical names, such as "glycine", or abbreviated symbols such as three letters, "Gly" or one letter "G. In this disclosure, both one letter and three letters will be used. Except glycine, all other common amino acids have stereoisomers, i.e., enantiomer, D or L form. The amino acids in most natural peptides and proteins are all in L-form. Some D-form amino acids are produced by microorganisms or present in antibiotics, and have inhibitory or antagonistic actions. For example, D-alanine, D-aspartic acid, and D-glutamic acid are present in bacterial cell walls, and D-glutamic acid, D-aspartic acid and D-phenylalanine are present in the antibiotic bacitracin. An uncommon amino acid is an amino acid that is not a common amino acid. Examples of uncommon amino acids include, but are not limited to, β-alanine and taurine. The uncommon amino acids can exist as a D or L form.

The one letter and three letter symbols used for the 20 common amino acids are as follows: alanine (A, Ala), arginine (R, Arg), aspartic acid (D, Asp), asparagine (N, Asn), cysteine (C, Cys), glycine (G, Gly), glutamic acid (E, Glu), glutamine (Q, Gln), histidine (H, His), isoleucine (I, Ile), leucine (L, Leu), lysine (K, Lys), methionine (M, Met), phenylalanine (F, Phe), proline (P, Pro), serine (S, Ser), threonine (T, Thr), tryptophan (W, Trp), tyrosine (Y, Tyr) and valine (V, Val).

The letter symbols used for uncommon amino acids are as follows: β-alanine (bAla), 4-aminobenzoic acid (Abz), 2-aminobutanoic acid (Abu), 4-aminobutanoic acid (4Abu), 2-aminoisobutanoic acid (Aib), 5-aminolevulinic acid (All), alliin (Ali), 2-aminoadipic acid (Aad), 3-aminoadipic acid (bAad), aminopimelic acid (Apa); 3-aminotyrosine (Atyr), canavanine (Cav), canaline (Can), ciliatine (Cil), cysteic acid (Cya), cysteine sulfinic acid (Csa), citruline (Cit); creatine (Cre), creatinine (Crn); 2,3-diaminosuccinic acid (Dsa); 2,4-diaminobutanoic acid (Dbu); 2,3-diaminopropanoic acid (Dpr); 3,4-dihydroxyphenyl-alanine (Dopa); 3,5-diiodotyrosine (Dtyr); homoarginine (Har), homoserine (Hser), homocysteine (Hcys), homocitrulline (Hcit), hydroxylysine (Hyl); 3-hydroxyproline (3Hyp); 4-hydroxyproline (4Hyp); 2-hydroxy-4-aminobutanoic acid (Haba); 3-hydroxy-4-aminobutanoic acid (Hyba); 4-hydroxyomithine (Horn); 4-hydroxyaspartic acid (Hasp); 4-hydroxyphenyl-glycine (Hpg); 3-iodotyrosine (Ityr), lanthionine (Lan), β-lysine (βLys); α-methylalanine (Mala); β-methylaspartic acid (Mas), 4-methylproline (Mpro); 2-methylserine (Mser); N-methylhistidine (Mhis); ornithine (Orn); phenylglycine (B or Pgly); 3-phenylserine (Pser); sarcosine (Sar); S-allyl-cysteine (Sac); theanine (The); thyroxine (Thy); 3,5,3'-triiodothyronine (Tth); and taurine (Tau).

The terms and abbreviations that can be used are as follows: acetyl, Ac; benzoyl, Bz; benzyl, Bzl; diphenylmethyl, Dpm; benzyl ester, OBzl; benzyloxycarbonyl, Z; t-butyl ester, OtBu; t-butyl, tBu; ethyl ester, OEt; formyl, For; hexyl ester, OHex; methyl ester, OMe; propanoyl, Pr; pyroglutamyl, Pyro; phenylacetyl, PhAc; and trityl, Trt.

A peptide bond, C(=O)NH, is a covalent bond formed between two amino acid molecules when the carboxyl group on one amino acid reacts with the amino group of the other amino acid in a dehydration synthesis reaction. A tripeptide is a peptide that contains three amino acid residues. Theoretically, about 8,000 different tripeptides can be formed from 20 common amino acids, and more than 300,000 different tripeptides can be formed from both the common and uncommon amino acids. A tetrapeptide is a peptide that contains four amino acid residues. A pentapeptide is a peptide that contains five amino acid residues. A hexapeptide is a peptide that contains six amino acid residues. A pentadecapeptide is a peptide that contains fifteen amino acid residues. A hexadecapeptide is a peptide that contains sixteen amino acid residues. A nonadecapeptide is a peptide that contains nineteen amino acid residues. An eicosapeptide is a peptide that contains twenty amino acid residues. Peptides can be further modified by substitutions, etc. Each peptide can have different chemical and physical properties, and has different biological and pharmacological actions.

When a particular group is "substituted", that group can have one or more substituents, preferably from one to five substituents, more preferably from one to three substituents, most preferably from one to two substituents, independently selected from the list of substituents.

With reference to substituents or amino acid residues in a peptide, the term "independently" means that when more than one of such substituents or amino acid residues are possible, such substituents or amino acid residues may be the same or different from each other.

As used herein, the term "subject" means any animal, preferably a mammal, most preferably a human, to whom will be or has been administered compounds or topical formulations according to embodiments of the invention. The term "mammal" as used herein, encompasses any mammal. Examples of mammals include, but are not limited to, cows, horses, sheep, pigs, cats, dogs, mice, rats, rabbits, guinea pigs, monkeys, humans etc., more preferably, a human.

"Treatment" or "treating" refers to amelioration, prophylaxis, or reversal of a disease or disorder, or at least one discernible symptom thereof. In another embodiment, "treatment" or "treating" refers to amelioration, prophylaxis, or reversal of at least one measurable physical parameter related to the disease or disorder being treated, not necessarily discernible in or by the mammal. In yet another embodiment, "treatment" or "treating" refers to inhibiting or slowing the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

In certain embodiments, compounds of interest are administered as a preventative measure. As used herein, "prevention" or "preventing" refers to a reduction of the risk of acquiring a given disease or disorder.

As used herein, a "therapeutically effective amount" of a compound of an embodiment of the present invention means the amount of the compound that elicits the biological or medicinal response in a tissue system, animal or human that is being sought by a researcher, veterinarian, medical doctor or other clinician, which includes alleviation of the symptoms of the disease or disorder being treated.

One skilled in the art will recognize that the therapeutically effective amount of a compound to be used in the instant invention can vary with factors, such as the particular subject, e.g., age, diet, health, etc., severity and complications and types of the symptom or disorder sought to be treated or prevented, the formulation used, etc.

In one aspect, an embodiment of the present invention relates to a composition for topical or systemic administration to a mammal comprising a pharmaceutically or cosmetically acceptable carrier and a therapeutically effective amount of a peptide derivative having the following generic Formula (I):

R₁AAB-(AAA)ₙ-AAC-R₂ Formula (I)

as defined in claim 1.

Based on Formula (I), illustrative N-acylpeptide derivatives that can be used in the present invention are set forth in the claims.

Representative N-acyltripeptide derivatives (n=1), are e.g.: N-Ac-Tyr-Tyr-Tyr-OH, N-Ac-Tyr-Tyr-Tyr-OEt, N-Ac-Tyr-Tyr-Tyr-NH₂, N-Ac-Tyr-Tyr-Tyr-NHAc, N-Ac-Tyr-Tyr-Tyr-NHNH₂, N-Ac-Tyr-Tyr-Tyr-NHNHAc, N-Ac-Cys-Cys-Tyr-NH₂, N-Ac-Cys-Tyr-Tyr-NH₂, N-Ac-Val-Val-Tyr-NH₂, N-Ae-Val-Tyr-Tyr-NH₂, N-Ac-Cys-Dopa-Tyr-NH₂, N-Ac-Dopa-Dopa-Tyr-NH₂, N-Ac-Dopa-Cys-Tyr-NH₂, N-Ac-Dopa-Dopa-Cys-NH₂, N-Ac-Tyr-Val-Ala-NH₂, N-Ac-Val-Ala-Tyr-NH₂, N-Ac-Glu-Cys-Ala-NH₂, N-Ac-Glu-Cys-Gly-NH₂, N-Ac-Asp-Cys-Gly-NH₂, N-Ac-Asp-Cys-Ala-NH₂, N-Ac-Tyr-Tyr-Tyr-NHOH, N-Ac-Val-Val-Ala-NHOH, N-Ac-Dopa-Dopa-Tyr-NHOH, N-Ac-Cys-Dopa-Tyr-NHOH, N-Pr-Tyr-Tyr-Tyr-NH₂, N-Pr-Tyr-Tyr-Tyr-NHAc, N-Pr-Tyr-Tyr-Tyr-NHNH₂, N-Pr-Tyr-Tyr-Tyr-NHNHAc, N-Pr-Cys-Cys-Tyr-NH₂, N-Pr-Dopa-Tyr-Tyr-NH₂, N-Pr-Dopa-Dopa-Tyr-NH₂, N-Pr-Cys-Dopa-Tyr-NH₂, N-Pr-Cys-Tyr-Tyr-NH₂, N-Pr-Tyr-Tyr-Tyr-NHOH, N-Pr-Val-Val-Ala-NHOH, N-Pr-Dopa-Dopa-Tyr-NHOH, N-Pr-Cys-Dopa-Tyr-NHOH, N-Bz-Tyr-Tyr-Tyr-NH₂, N-Bz-Tyr-Tyr-Tyr-NHNH₂, N-Bz-Tyr-Tyr-Tyr-NHNHAc, N-Bz-Cys-Cys-Tyr-NH₂, N-Bz-Dopa-Tyr-Tyr-NH₂, N-Bz-Dopa-Dopa-Tyr-NH₂, N-Bz-Cys-Dopa-Tyr-NH₂, N-Bz-Cys-Tyr-Tyr-NH₂, N-Bz-Tyr-Tyr-Tyr-NHOH, N-Bz-Val-Val-Ala-NHOH, N-Bz-Dopa-Dopa-Tyr-NHOH, and N-Bz-Cys-Dopa-Tyr-NHOH.

Other N-acyltripeptide derivatives are:
N-Ac-Tyr-Tyr-Tyr-OH, N-Ac-Tyr-Tyr-Tyr-OEt, N-Ac-Tyr-Tyr-Tyr-NH₂, N-Ac-Cys-Cys-Tyr-NH₂, N-Ac-Cys-Tyr-Tyr-NH₂, N-Ac-Val-Val-Tyr-NH₂, N-Ac-Val-Tyr-Tyr-NH₂, N-Ac-Cys-Dopa-Tyr-NH₂, N-Ac-Dopa-Dopa-Tyr-NH₂, N-Ac-Dopa-Cys-Tyr-NH₂, N-Ac-Dopa-Dopa-Cys-NH₂, N-Ac-Tyr-Val-Ala-NH₂, N-Ac-Val-Ala-Tyr-NH₂, N-Pr-Tyr-Tyr-Tyr-NH₂, N-Pr-Cys-Cys-Tyr-NH₂, N-Pr-Dopa-Tyr-Tyr-NH₂, N-Pr-Dopa-Dopa-Tyr-NH₂, N-Pr-Cys-Dopa-Tyr-NH₂, N-Pr-Cys-Tyr-Tyr-NH₂, N-Bz-Tyr-Tyr-Tyr-NH₂, N-Bz-Cys-Cys-Tyr-NH₂, N-Bz-Dopa-Tyr-Tyr-NH₂, N-Bz-Dopa-Dopa-Tyr-NH₂, N-Bz-Cys-Dopa-Tyr-NH₂, and N-Bz-Cys-Tyr-Tyr-NH₂.

Further N-acyltripeptide derivatives are:
N-Ac-Tyr-Tyr-Tyr-NH₂, N-Ac-Cys-Cys-Tyr-NH₂, N-Ac-Cys-Tyr-Tyr-NH₂, N-Ac-Cys-Dopa-Tyr-NH₂, N-Ac-Dopa-Dopa-Tyr-NH₂, N-Ac-Dopa-Cys-Tyr-NH₂, N-Ac-Dopa-Dopa-Cys-NH₂, N-Ac-Tyr-Val-Ala-NH₂, N-Ac-Val-Ala-Tyr-NH₂, N-Pr-Tyr-Tyr-Tyr-NH₂, N-Pr-Cys-Cys-Tyr-NH₂, N-Pr-Dopa-Tyr-Tyr-NH₂, N-Pr-Dopa-Dopa-Tyr-NH₂, N-Pr-Cys-Dopa-Tyr-NH₂, N-Pr-Cys-Tyr-Tyr-NH₂, and N-Bz-Tyr-Tyr-Tyr-NH₂.

N-acyltetrapeptide derivatives (n=2), are e.g. :
N-Ac-Tyr-Tyr-Tyr-Tyr-OH, N-Ac-Tyr-Tyr-Tyr-Tyr-OEt, N-Ac-Tyr-Tyr-Tyr-Tyr-NH₂, N-Ac-Tyr-Tyr-Tyr-Tyr-NHAc, N-Ac-Tyr-Tyr-Tyr-Tyr-NHNH₂, N-Ac-Tyr-Tyr-Tyr-Tyr-NHNHAc, N-Ac-Tyr-Tyr-Tyr-Tyr-NHOH, N-Pr-Tyr-Tyr-Tyr-Tyr-OH, N-Pr-Tyr-Tyr-Tyr-Tyr-OEt, N-Pr-Tyr-Tyr-Tyr-Tyr-NH₂, N-Pr-Tyr-Tyr-Tyr-Tyr-NHPr, N-Pr-Tyr-Tyr-Tyr-Tyr-NHNH₂, N-Pr-Tyr-Tyr-Tyr-Tyr-NHNHPr, N-Pr-Tyr-Tyr-Tyr-Tyr-NHOH, N-Bz-Tyr-Tyr-Tyr-Tyr-OH, N-Bz-Tyr-Tyr-Tyr-Tyr-OEt, N-Bz-Tyr-Tyr-Tyr-Tyr-NH₂, N-Bz-Tyr-Tyr-Tyr-Tyr-NHBz, N-Bz-Tyr-Tyr-Tyr-Tyr-NHNH₂, N-Bz-Tyr-Tyr-Tyr-Tyr-NHNHBz, and N-Bz-Tyr-Tyr-Tyr-Tyr-NHOH (SEQ ID NO: 7- SEQ ID NO: 27, respectively).

N-acylpentapeptide derivatives (n=3), are e.g.:
N-Ac-Tyr-Tyr-Tyr-Tyr-Tyr-OH, N-Ac-Tyr-Tyr-Tyr-Tyr-Tyr-OEt, N-Ac-Tyr-Tyr-Tyr-Tyr-Tyr-NH₂, N-Ac-Tyr-Tyr-Tyr-Tyr-Tyr-NHAc, N-Ac-Tyr-Tyr-Tyr-Tyr-Tyr-NHNH₂, N-Ac-Tyr-Tyr-Tyr-Tyr-Tyr-NHNHAc, N-Ac-Tyr-Tyr-Tyr-Tyr-Tyr-NHOH, N-Pr-Tyr-Tyr-Tyr-Tyr-Tyr-OH, N-Pr-Tyr-Tyr-Tyr-Tyr-Tyr-OEt, N-Pr-Tyr-Tyr-Tyr-Tyr-Tyr-NH₂, N-Pr-Tyr-Tyr-Tyr-Tyr-Tyr-NHPr, N-Pr-Tyr-Tyr-Tyr-Tyr-Tyr-NHNH₂, N-Pr-Tyr-Tyr-Tyr-Tyr-Tyr-NHNHPr, N-Pr-Tyr-Tyr-Tyr-Tyr-Tyr-NHOH, N-Ac-Gly-Ile-Arg-Val-Ala-OH, N-Ac-Gly-Ile-Arg-Val-Ala-OEt, N-Ac-Gly-Ile-Arg-Val-Ala-NH₂, N-Ac-Gly-Ile-Arg-Val-Ala-NHAc, N-Ac-Gly-Ile-Arg-Val-Ala-NHNH₂, N-Ac-Gly-Ile-Arg-Val-Ala-NHNHAc, N-Ac-Gly-Ile-Arg-Val-Ala-NHOH, N-Ac-Ala-Leu-Lys-His-Arg-OH, N-Ac-Ala-Leu-Lys-His-Arg-OEt, N-Ac-Ala-Leu-Lys-His-Arg-NH₂, N-Ac-Ala-Leu-Lys-His-Arg-NHAc, N-Ac-Ala-Leu-Lys-Plis-Arg-NHNH₂, N-Ac-Ala-Leu-Lys-His-Arg-NHNHAc, N-Ac-Ala-Leu-Lys-His-Arg-NHOH, N-Pr-Ala-Leu-Lys-His-Arg-OH, N-Pr-Ala-Leu-Lys-His-Arg-OEt, N-Pr-Ala-Leu-Lys-His-Arg-NH₂, N-Pr-Ala-Leu-Lys-His-Arg-NHPr, N-Pr-Ala-Leu-Lys-His-Arg-NHNH₂, N-Pr-Ala-Leu-Lys-His-Arg-NHNHPr, N-Pr-Ala-Leu-Lys-His-Arg-NHOH,
N-Bz-Ala-Leu-Lys-His-Arg-OH, N-Bz-Ala-Leu-Lys-His-Arg-OEt, N-Bz-Ala-Leu-Lys-His-Arg-NH₂, N-Bz-Ala-Leu-Lys-His-Arg-NHBz, N-Bz-Ala-Leu-Lys-His-Arg-NHNH₂, N-Bz-Ala-Leu-Lys-His-Arg-NHNHPr, and N-Bz-Ala-Leu-Lys-His-Arg-NHOH (SEQ ID NO: 28- SEQ ID NO: 69, respectively).

Further N-acylpentapeptide derivatives are:
N-Ac-Tyr-Tyr-Tyr-Tyr-Tyr-OH, N-Ac-Tyr-Tyr-Tyr-Tyr-Tyr-OEt, N-Ac-Tyr-Tyr-Tyr-Tyr-Tyr-NH₂, N-Pr-Tyr-Tyr-Tyr-Tyr-Tyr-OH, N-Pr-Tyr-Tyr-Tyr-Tyr-Tyr-OEt, N-Pr-Tyr-Tyr-Tyr-Tyr-Tyr-NH₂, N-Ac-Ala-Leu-Lys-His-Arg-OH, N-Ac-Ala-Leu-Lys-His-Arg-OEt, N-Ac-Ala-Leu-Lys-His-Arg-NH₂, N-Ac-Ala-Leu-Lys-His-Arg-NHAc, N-Ac-Ala-Leu-Lys-His-Arg-NHOH, N-Pr-Ala-Leu-Lys-His-Arg-OH, N-Pr-Ala-Leu-Lys-His-Arg-OEt, N-Pr-Ala-Leu-Lys-His-Arg-NH₂, N-Pr-Ala-Leu-Lys-His-Arg-NHPr, N-Bz-Ala-Leu-Lys-His-Arg-OH, N-Bz-Ala-Leu-Lys-His-Arg-OEt, N-Bz-Ala-Leu-Lys-His-Arg-NH₂, and N-Bz-Ala-Leu-Lys-His-Arg-NHBz (SEQ ID NOs: 28-30, 35-37, 49-52, 55-59, and 63-66, respectively.)

Further N-acylpentapeptide derivatives are:
N-Ac-Tyr-Tyr-Tyr-Tyr-Tyr-NH₂, N-Pr-Tyr-Tyr-Tyr-Tyr-Tyr-NH₂, N-Ac-Ala-Leu-Lys-His-Arg-OH, N-Ac-Ala-Leu-Lys-His-Arg-OEt, N-Ac-Ala-Leu-Lys-His-Arg-NH₂, N-Ac-Ala-Leu-Lys-His-Arg-NHAc, N-Pr-Ala-Leu-Lys-His-Arg-OH, N-Pr-Ala-Leu-Lys-His-Arg-OEt, N-Pr-Ala-Leu-Lys-His-Arg-NH₂, N-Pr-Ala-Leu-Lys-His-Arg-NHPr, N-Bz-Ala-Leu-Lys-His-Arg-OH, N-Bz-Ala-Leu-Lys-His-Arg-OEt, and N-Bz-Ala-Leu-Lys-His-Arg-NH₂ (SEQ ID NOs: 30, 37, 49, 50, 51, 52, 56, 57, 58, 59, 63, 64, and 65, respectively).

Representative N-acylhexapeptide derivatives (n=4), are:
N-Ac-Tyr-Tyr-Tyr-Tyr-Tyr-Tyr-OH, N-Ac-Tyr-Tyr-Tyr-Tyr-Tyr-Tyr-OEt, N-Ac-Tyr-Tyr-Tyr-Tyr-Tyr-Tyr-NH₂, N-Ac-Tyr-Tyr-Tyr-Tyr-Tyr-Tyr-NHAc, N-Ac-Tyr-Tyr-Tyr-Tyr-Tyr-Tyr-NHNH₂, N-Ac-Tyr-Tyr-Tyr-Tyr-Tyr-Tyr-NHNHAc, N-Ac-Tyr-Tyr-Tyr-Tyr-Tyr-Tyr-NHOH, N-Pr-Tyr-Tyr-Tyr-Tyr-Tyr-Tyr-OH, N-Pr-Tyr-Tyr-Tyr-Tyr-Tyr-Tyr-OEt, N-Pr-Tyr-Tyr-Tyr-Tyr-Tyr-Tyr-NH₂,N-Pr-Tyr-Tyr-Tyr-Tyr-Tyr-Tyr-NHPr,N-Pr-Tyr-Tyr-Tyr-Tyr-Tyr-Tyr-NHNH₂, N-Pr-Tyr-Tyr-Tyr-Tyr-Tyr-Tyr-NHNHPr, N-Pr-Tyr-Tyr-Tyr-Tyr-Tyr-Ty-r-NHOH, N-Bz-Tyr-Tyr-Tyr-Tyr-Tyr-Tyr-OH, N-Bz-Tyr-Tyr-Tyr-Tyr-Tyr-Tyr-OEt, N-Bz-Tyr-Tyr-Tyr-Tyr-Tyr-Tyr-NH₂, N-Bz-Tyr-Tyr-Tyr-Tyr-Tyr-Tyr-NHBz, N-Bz-Tyr-Tyr-Tyr-Tyr-Tyr-Tyr-NHNH₂, N-Bz-Tyr-Tyr-Tyr-Tyr-Tyr-Tyr-NHNHBz, N-Bz-Tyr-Tyr-Tyr-Tyr-Tyr-Tyr-NHOH, N-Ac-Cys-Ser-Val-Thr-Cys-Gly-OH, N-Ac-Cys-Ser-Val-Thr-Cys-Gly-OEt, N-Ac-Cys-Ser-Val-Thr-Cys-Gly-NH₂, N-Ac-Cys-Ser-Val-Thr-Cys-Gly-NHAc, N-Ac-Cys-Ser-Val-Thr-Cys-Gly-NHNH₂, N-Ac-Cys-Ser-Val-Thr-Cys-Gly-NHNHAc, N-Pr-Cys-Ser-Val-Thr-Cys-Gly-OH, N-Pr-Cys-Ser-Val-Thr-Cys-Gly-OEt, N-Pr-Cys-Ser-Val-Thr-Cys-Gly-NH₂, N-Pr-Cys-Ser-Val-Thr-Cys-Gly-NHPr, N-Pr-Cys-Ser-Val-Thr-Cys-Gly-NHNH₂, and N-Pr-Cys-Ser-Val-Thr-Cys-Gly-NHNHPr (SEQ ID NO: 70- SEQ ID NO: 102, respectively).

N-acylpentadecapeptide derivatives (n=13), are for example:
N-Ac-(EASPEAVAGVGFESK)-OH, N-Ac-(EASPEAVAGVGFESK)-OEt, N-Ac-(EASPEAVAGVGFESK)-NH₂, N-Ac-(EASPEAVAGVGFESK)-NHAc, N-Ac-(EASPEAVAGVGFESK)-NHNH₂, N-Ac-(EASPEAVAGVGFESK)-NHNHAc, N-Ac-(EASPEAVAGVGFESK)-NHOH, N-Pr-(EASPEAVAGVGFESK)-OH, N-Pr-(EASPEAVAGVGFESK)-OEt, N-Pr-(EASPEAVAGVGFESK)-NH₂, N-Pr-(EASPEAVAGVGFESK)-NHPr, N-Pr-(EASPEAVAGVGFESK)-NHNH₂, N-Pr-(EASPEAVAGVGFESK)-NHNHPr, N-Pr-(EASPEAVAGVGFESK)-NHOH, N-Bz-(EASPEAVAGVGFESK)-OH, N-Bz-(EASPEAVAGVGFESK)-OEt, N-Bz-(EASPEAVAGVGFESK)-NH₂, N-Bz-(EASPEAVAGVGFESK)-NMBz, N-Bz-(EASPEAVAGVGFESK)-NHNH₂, N-Bz-(EASPEAVAGVGFESK)-NHNHBz, and N-Bz-(EASPEAVAGVGFESK)-NHOH, (SEQ ID NO: 103- SEQ ID NO: 123, respectively).

Representative N-acylhexadecapeptide derivatives (n=14), are e.g.:
N-Ac-(EEASPEAVAGVGFESK)-OH, N-Ac-(EEASPEAVAGVGFESK)-OEt, N-Ac-(EEASPEAVAGVGFESK)-NH₂, N-Ac-(EEASPEAVAGVGFESK)-NHAc, N-Ac-(EEASPEAVAGVGFESK)-NHNH₂, N-Ac-(EEASPEAVAGVGFESK)-NHNHAc, N-Ac-(EEASPEAVAGVGFESK)-NHOH, N-Pr-(EEASPEAVAGVGFESK)-OH, N-Pr-(EEASPEAVAGVGFESK)-OEt, N-Pr-(EEASPEAVAGVGFESK)-NH₂, N-Pr-(EEASPEAVAGVGFESK)-NHPr, N-Pr-(EEASPEAVAGVGFESK)-NHNH₂, N-Pr-(EEASPEAVAGVGFESK)-NHNHPr, N-Pr-(EEASPEAVAGVGFESK)-NHOH, N-Bz-(EEASPEAVAGVGFESK)-OH, N-Bz-(EEASPEAVAGVGFESK)-OEt, N-Bz-(EEASPEAVAGVGFESK)-NH₂, N-Bz-(EEASPEAVAGVGFESK)-NHBz, N-Bz-(EEASPEAVAGVCFESK)-NHNH₂, N-Bz-(EEASPEAVAGVGFESK)-NHNHBz, N-Bz-(EEASPEAVAGVGFESK)-NHOH, N-Ac-(EEASPEAVAGVGBESK)-NH₂, and N-Ac-(EEASPEAVAGVGBESK)-NHNH₂, (SEQ ID NO: 124- SEQ ID NO: 146, respectively), wherein"B" representing "Pgly".

Further N-acylhexadecapeptide derivatives are:
N-Ac-(EEASPEAVAGVGFESK)-OH, N-Ac-(EEASPEAVAGVGFESK)-OEt, N-Ac-(EEASPEAVAGVGFESK)-NH₂, N-Ac-(EEASPEAVAGVGFESK)-NHAc, N-Pr-(EEASPEAVAGVGFESK)-OH, N-Pr-(EEASPEAVAGVGFESK)-OEt, N-Pr-(EEASPEAVAGVGFESK)-NH₂, N-Pr-(EEASPEAVAGVGFESK)-NHPr, N-Bz-(EEASPEAVAGVGFESK)-OH, N-Bz-(EEASPEAVAGVGFESK)-OEt, N-Bz-(EEASPEAVAGVGFESK)-NH₂, N-Bz-(EEASPEAVAGVGFESK)-NHBz, N-Ac-(EEASPEAVAGVGBESK)-NH₂ and N-Ac-(EEASPEAVAGVGBESK)-NHNH₂ (SEQ ID NOs: 124, 125, 126, 127, 131, 132, 133, 134, 138, 139, 140, 141, 145, and 146, respectively).

Representative N-acylnonadecapeptide derivatives (n=17), are e.g.:
N-Ac-(CKKEEASPEAVAGVGFESK)-OH, N-Ac-(CKKEEASPEAVAGVGFESK)-OEt, N-Ac-(CKKEEASPEAVAGVGFESK)-NH₂, N-Ac-(CKKEEASPEAVAGVGFESK)-NHAc, N-Ac-(CKKEEASPEAVAGVGFESK)-NHNH₂, N-Ac-(CKKEEASPEAVAGVGFESK)-NHNHAc, and N-Ac-(CKKEEASPEAVAGVGFESK)-NHOH, (SEQ ID NO: 147- SEQ ID NO: 153, respectively).

Representative N-acyleicosapeptide derivatives (n=18), are e.g.:
N-Ac-(FCTGIRVAHLALKHRQGKNH)-OH, N-Ac-(FCTGIRVAHLALKHRQGKNH)-OEt, N-Ac-(FCTGIRVAHLALKHRQGKNH)-NH₂, N-Ac-(FCTGIRVAHLALKHRQGKNH)-NHAc,
N-Ac-(FCTGIRVAHLALKHRQGKNH)-NHNH₂, N-Ac-(FCTGIRVAHLALKHRQGKNH)-NHNHAc, N-Ac-(FCTGIRVAHLALKHRQGKNH)-NHOH, N-Pr-(FCTGIRVAHLALKHRQGKNH)-OH, N-Pr-(FCTGIRVAHLALKHRQGKNH)-OEt,
N-Pr-(FCTGIRVAHLALKHRQGKNH)-NH₂, N-Pr-(FCTGIRVAHLALKHRQGKNH)-NHPr, N-Pr-(FCTGIRVAHLALKHRQGKNH)-NHNH₂, N-Pr-(FCTGIRVAHLALKHRQGKNH)-NHNHPr, N-Pr-(FCTGIRVAHLALKHRQGKNH)-NHOH, N-Bz-(FCTGIRVAHLALKHRQGKNH)-OH, N-Bz-(FCTGIRVAHLALKHRQGKNH)-OEt,
N-Bz-(FCTGIRVAHLALKHRQGKNH)-NH₂, N-Bz-(FCTGIRVAHLALKHRQGKNH)-NHBz, N-Bz-(FCTGIRVAHLALKHRQGKNH)-NHNH₂, N-Bz-(FCTGIRVAHLALKHRQGKNH)-NHNHBz, and N-Bz-(FCTGIRVAHLALKHRQGKNH)-NHOH, (SEQ ID NO: 154- SEQ ID NO: 174, respectively).

An N-acylpeptide derivative may be seclected from the group consisting of: N-Ac-Tyr-Tyr-Tyr-OH, N-Ac-Tyr-Tyr-Tyr-OEt, N-Ac-Tyr-Tyr-Tyr-NH₂, N-Ac-Tyr-Tyr-Tyr-NHAc, N-Ac-Tyr-Tyr-Tyr-NHNH₂, N-Ac-Tyr-Tyr-Tyr-NHNHAc, N-Ac-Tyr-Tyr-Tyr-NHOH, N-Ac-Tyr-Tyr-Tyr-Tyr-OH, N-Ac-Tyr-Tyr-Tyr-Tyr-OEt, N-Ac-Tyr-Tyr-Tyr-Tyr-NH₂, N-Ac-Tyr-Tyr-Tyr-Tyr-NHAc, N-Ac-Tyr-Tyr-Tyr-Tyr-NHNH₂, N-Ac-Tyr-Tyr-Tyr-Tyr-NHNHAc, N-Ac-Tyr-Tyr-Tyr-Tyr-NHOH, N-Ac-Tyr-Tyr-Tyr-Tyr-Tyr-OH, N-Ac-Tyr-Tyr-Tyr-Tyr-Tyr-OEt, N-Ac-Tyr-Tyr-Tyr-Tyr-Tyr-NH₂, N-Ac-Tyr-Tyr-Tyr-Tyr-Tyr-NHAc, N-Ac-Tyr-Tyr-Tyr-Tyr-Tyr-NHNH₂,N-Ac-Tyr-Tyr-Tyr-Tyr-Tyr-NHNHAc,N-Ac-Tyr-Tyr-Tyr-Tyr-Tyr-NHOH,
N-Ac-Ala-Leu-Lys-His-Arg-OH, N-Ac-Ala-Leu-Lys-His-Arg-OEt, N-Ac-Ala-Leu-Lys-His-Arg-NH₂, N-Ac-Ala-Leu-Lys-His-Arg-NHAc. N-Ac-Ala-Leu-Lys-His-Arg-NHNH₂, N-Ac-Ala-Leu-Lys-His-Arg-NHNHAc, N-Ac-Ala-Leu-Lys-His-Arg-NHOH, N-Ac-Tyr-Tyr-Tyr-Tyr-Tyr-Tyr-OH,
N-Ac-Tyr-Tyr-Tyr-Tyr-Tyr-Tyr-OEt, N-Ac-Tyr-Tyr-Tyr-Tyr-Tyr-Tyr-NH₂,
N-Ac-(EEASPEAVAGVGFESK)-OH, N-Ac-(EEASPEAVAGVGFESK)-OEt, N-Ac-(EEASPEAVAGVGFESK)-NH₂, N-Ac-(EEASPEAVAGVGFESK)-NHAc, N-Ac-(EEASPEAVAGVGFESK)-NHNH₂, N-Ac-(EEASPEAVAGVGFESK)-NHNHAc, N-Ac-(EEASPEAVAGVGFESK)-NHOH, N-Ac-(EEASPEAVAGVGBESK)-NH₂, and N-Ac-(EEASPEAVAGVGBESK)-NHNH₂ (SEQ ID NOs: 7, 8, 9, 10, 11, 12, 13,28, 29, 30, 31, 32, 33, 34, 49, 50, 51, 52, 53, 54, 55, 70, 71, 72, 124, 125, 126, 127, 128, 129, 130, 145, and 146, respectively).

N-acylpeptide derivatives according to embodiments of the present invention can be made by any method known to those skilled in the art in view of the present disclosure.

Chemical and physical properties, biological functions and therapeutic effects of a peptide depend exclusively on the nature and sequence of amino acid residues, and different amino acid residues or different amino acid sequences may result in a completely different peptide. In addition to chemical and physical properties, biological functions and therapeutic effects of a peptide are also changed when the functional groups of such peptides are modified by substitution. In most cases, the N-acylpeptide derivatives of the present invention have different and much improved chemical and physical properties, biological functions and therapeutic effects as compared to an unmodified peptide.

A peptide is usually an amphoteric substance, having positive and negative charges in the same molecule. A peptide normally cannot penetrate the skin on topical application because of the tough stratum corneum layer acting as a permeation barrier. In general, an ionic substance or any substance with a molecular weight of more than 800 daltons cannot readily penetrate the intact skin. The N-acylpeptide derivatives of the present invention have the alkaline radical such as an amino group modified by acylation, so that they are no longer amphoteric in nature, and are readily bioavailable for penetration and/or distribution to target tissues or sites for pharmacological actions by topical or systemic administration.

Another general aspect of the present invention relates to a method of treating or preventing a disease, symptom or syndrome associated with tumors, cancers, immune, nervous, vascular, musculoskeletal, cutaneous system, or other tissues or systems in a subject in need of treatment. The method comprises topically or systemically administering to the subject a composition comprising a therapeutically effective amount of an N-acylpeptide derivative according to an embodiment of the present invention and a pharmaceutically or cosmetically acceptable carrier.

Conditions, disorders, symptoms and syndromes associated with the (A) tumors and cancers, (B) immune system, (C) nervous system, (D) vascular system, (E) musculoskeletal system, (F) cutaneous system, and (G) other tissues or systems that can be treated with a composition of the present invention are described as follows.

### (A) Tumors and Cancers.

Cancer is an unregulated proliferation of cells due to loss of normal controls, resulting in abnormal growth, lack of differentiation, local tissue invasion, and often, metastasis. Tumor is an abnormal growth of cells or tissues which may be benign or malignant. Tumors or cancers that can be treated with a composition of the present invention include, but are not limited to, actinic keratosis, adrenal cancer, basal cell carcinoma, bladder cancer, brain tumor, breast cancer, cervical cancer, colon cancer, esophagus cancer, head and neck cancer, Hodgkin disease, Kaposi's sarcoma, larynx cancer, leukemia, lung carcinoma, liver cancer, melanoma, multiple myeloma, mesothelioma, ovarian cancer, pancreatic cancer, prostate cancer, renal cancer, rectal cancer, stomach cancer, squamous cell carcinoma, thyroid cancer, testicular cancer, thyroid cancer, and uterine cancer. Breast cancer most often involves glandular breast cells in the ducts or lobules, and can invade locally and spread through lymph nodes and into the bloodstream, then to lungs, liver, bone, brain and skin. Lung carcinoma is a leading cause of lung cancer with symptoms of coughing, chest discomfort or pain, and weight loss. Liver cancer is usually hepatocellular carcinoma often resulting from liver cirrhosis. Pancreatic cancer, primarily ductal adenocarcinoma has symptoms of weight loss, abdominal pain, and jaundice. Brain tumors such as gliomas, medulloblastomas and ependymomas can have symptom of headache, pain, edema, etc.

The development and growth of tumors and cancers can be due to deranged immune system even though the tumors or cancers may be caused by mutations.

### (B) Immune System.

The immune system, very similar to organs such as liver, kidney and thyroid, is composed of specialized cells that play a vital role in host defense. These cells include leukocytes (white blood cells) and dendritic cells. The leukocytes are divided into granulocytes (65%); specific granules in the cytoplasm such as neutrophils, eosinophils, and basophils; and agranulocytes; no specific granules in the cytoplasm such as lymphocytes (25-35%) and monocytes (5-10%). The lymphocytes are subdivided into B lymphocytes (antibody production) and T lymphocytes (foreign agent and tissue destruction). The monocyte can migrate from blood to tissue, and becomes macrophage. The dendric cell is derived from bone marrow and is critical in activation and priming of lymphocyte.

Deranged immune system can cause the following disorders:
(1) Rheumatic, connective tissue or collagen diseases. These diseases include, but are not limited to, systemic lupus erythematosus, rheumatoid arthritis, seronegative spondylarthritides (ankylosing spondylitis), Sjogren's syndrome (keratoconjunctivitis sicca, xerostomia), systemic sclerosis, polymyositis and dermatomyositis.
(2) Endocrine autoimmune diseases. These diseases include, but are not limited to, Type 1 diabetes, autoimmune thyroid disease such as Graves' disease and Hashimoto's thyroiditis, and Addison's disease.
(3) Liver diseases. These diseases include, but are not limited to, autoimmune hepatitis, sclerosing cholangitis, biliary cirrhosis, viral hepatitis including hepatitis A, hepatitis B, and hepatitis C.
(4) Gastrointestinal diseases. These diseases include, but are not limited to, mucosal disorder, atrophic gastritis, pernicious anemia, inflammatory bowel disease, and allergic food reactions.
(5) Immune mediated nephritis and vasculitis. These diseases include, but are not limited to, glomerulonephritis, Wegener's granulomatosis, microscopic polyarteritis, and cryoglobulinanemia.
(6) Immune mediated skin diseases. These diseases include, but are not limited to, psoriasis, vitiligo, bullous pemphigoid, pemphigus vulgaris, and pemphigus foliaceus.
(7) Immune mediated diseases of nervous system and eye. These diseases include, but are not limited to, multiple sclerosis, Guillain-Barre syndrome, myasthenia gravis, Lambert-Eaton syndrome, stiff man syndrome, keratitis, keratoconjunctivitis sicca, scleritis, episcleritis, and uveitis.
(8) Human immunodeficiency virus (HIV) and acquired immune deficiency syndrome (AIDS). HIV is a member of retrovirus family, with a single-stranded RNA genome. Such RNA genome can encode the enzyme reverse transcriptase, capable of transcribing viral RNA into DNA, and allowing the virus to integrate into the host cell genome. During the initial stage of infection, the virus targets memory CD4 T lymphocytes as a receptor, and depletes CD4 T cells from gut and peripheral lymph nodes. The immunity from B lymphocytes, dendritic cells and macrophages is also weakened. The vaccine remains the best hope of controlling HIV infection, however, there are numerous issues to be resolved for an effective, inexpensive and safe immunization against HIV infection. The challenging issues are (a) the virus can survive and be transmitted within a host and between hosts in extracellular form, as blood borne virus particles, and also in intracellular form hidden within infected host cells, (b) the virus copies its genome into host cells, and a live attenuated virus vaccine may pose safety issues, (c) the virus has multiple strains and a very high mutability which is challenging for a vaccine using fixed virus sequences, and may not be effective for other strains, (d) there is no small animal model existing for HIV infection, and the efficacy studies carried out for non-human primates are rather expensive.

Other deranged immune system may also involve the growth and spread (metastasis) of tumors and cancers.

### (C) Nervous System.

The conditions, disorders, symptoms and syndromes associated with the nervous system include, but are not limited to, the following conditions or disorders, which may present as indicated, or otherwise: (1) dementia and Alzheimer's disease: progressive loss of memory, shrinkage and atrophy of cerebral cortex, tangles of fibers in nerve cells, senile plaques of β-amyloid, decreased choline acetyltransferase enzyme; (2) carpal tunnel syndrome: weakness, pain, tingling, numbness, burning in palm and fingers; (3) encephalitis: inflammation of the brain; (4) headache: migraine, expansion of blood vessels pressing on nerves or constriction blocking blood supply, inflammation, muscle contraction to face, neck or scalp; (5) meningitis: infection of spinal fluid and meninges; (6) neuralgia: nerve pain, peripheral neuropathy, sciatica, shingles, trigeminal neuralgia; (7) Parkinson's disease: tremors in limbs, muscular rigidity; (8) amnesia: loss of memory and inability to form new memory; and (9) others, such as nervousness, anxiety, ataxia, Bell's palsy, epilepsy, multiple sclerosis, myasthenia gravis, narcolepsy, paralysis and rabies.

Alzheimer's disease causes progressive cognitive deterioration and is characterized by senile plaques of β-amyloid deposits, neurofibrillary tangles in the cerebral cortex and subcortical gray matter, and currently there is no cure.

Parkinson's disease is an idiopathic, slowly progressive, degenerative central nerve system (CNS) disorder characterized by resting tremor, muscular rigidity, slow and decreased movement, and postural instability, and currently there is no cure.

### (D) Vascular System.

The vascular conditions, reactions and disorders that can be treated with a composition of the present invention include, but are not limited to, acanthosis nigricans, acrocyanosis, actinic cheilitis, actinic prurigo, dermatitis, dermatosis, dermographism, dyshidrosis, drug eruptions, inflammation, or eczema, erythema, erythema migrans, erythrocyanosis, erythromelalgia, familial hemorrhage, histamine reaction, hypertension, inflammatory papular and pustular lesions, lichen planus, lupus erythematosus, mycosis fungoides, neurodermatitis, neuropeptide and neurovascular reactions, parapsoriasis, perniosis (chilblains), photoallergy, photoreaction, photosensitivity, pityriasis rosea, pityriasis rubra pilaris, polymorphic light eruption, psoriasis, rhinophyma, rosacea, sclerosis, spider naevi, T-cell disorders, telangiectasia, varicose veins (varicosis), urticaria, vessel dilation, and other vascular reactions.

### (E) Musculoskeletal System.

The conditions or abnormalities of musculoskeletal system include, but are not limited to, the following conditions or disorders, which may present as indicated, or otherwise: (1) osteoporosis: reduction of calcium in bone leading to thin bone and bone susceptible to fracture; (2) osteoarthritis: inflammation of joint cartilage provoking swelling and pain; (3) rheumatoid arthritis: inflammation of synovium and destruction of cartilage, damage to heart, lungs, nerves and eyes; (4) ankylosing spondylitis: arthritis affecting sacroiliac joints and spine with inflammation and immovability; (5) bursitis: inflammation of bursa; (6) tendinitis: inflammation of tendon; (7) gout: recurrent acute arthritis from uric acid deposit; and (8) specifically, neck, shoulder, elbow, wrist, lower back, hip, knee and ankle pains, inflammation, and arthritis.

### (F) Cutaneous System.

The cosmetic, dermatological or other conditions and disorders of cutaneous system that can be treated with a composition of the present invention include, but are not limited to, infections, deranged or disordered cutaneous or mucocutaneous tissue relevant to skin, nail and hair; oral, vaginal and anal mucosa; disturbed keratinization; inflammation; changes associated with intrinsic and extrinsic aging, and others which may or may not be related to cutaneous system. The manifestations include, but are not limited to: oily skin; acne; rosacea; age spots; blemished skin; blotches; cellulite; dermatoses; dermatitis; skin, nail and hair infections; dandruff; dryness or looseness of skin, nail and hair; xerosis; inflammation, or eczema; elastosis; herpes; hyperkeratosis; hyperpigmented skin; ichthyosis; keratoses; lentigines; melasmas; mottled skin; pseudofolliculitis barbae; photoaging and photodamage; pruritus; psoriasis; skin lines; stretch marks; thinning of skin, nail plate and hair; warts; wrinkles; oral or gum disease; irritated, inflamed, red, unhealthy, damaged or abnormal mucosa, skin, hair, nail, nostril, ear canal, anal or vaginal conditions; breakdown, defective synthesis or repair of dermal components; abnormal or diminished synthesis of collagen, glycosaminoglycans, proteoglycans and elastin, as well as diminished levels of such components in the dermis; uneven skin tone; uneven and rough surface of skin, nail and hair; loss or reduction of skin, nail and hair resiliency, elasticity and recoilability; laxity; lack of skin, nail and hair lubricants and luster; fragility and splitting of nail and hair; yellowing skin; reactive, irritating or telangiectatic skin; and dull and older-looking skin, nail and hair. In addition, the composition of the current invention can be used for general care of skin, nail and hair; to improve skin texture and pores, flakiness and redness; to make skin soft, smooth, fresh, balanced, visibly clear, even-toned and brighter; to increase skin fullness and plumpness; and for skin bleach and lightening and wound healing; to reduce or prevent sweating or perspiration of underarm, crotch, palm, or other parts of the body.

Skin, nail and hair infections can be caused by microorganisms which include bacteria, fungi, yeasts, molds, parasites and viruses. More specifically, the bacterial infections can cause trichomycosis axillaris, pitted keratolysis, erythrasma, impetigo, ecthyma, furunculosis (boils), carbuncle, scalded skin syndrome, toxic shock syndrome, erysipelas, cellulitis, necrotizing fasciitis, erysipeloid, cat-scratch disease (*Rochalimaea henselae)*, syphilis, lyme disease *(Borrelia burgdorferi),* cutaneous anthrax *(Bacillus anthracis),* gonococcal septicaemia, inoculation tuberculosis, scrofuloderma, tuberculides, erythema induratum, leprosy *(Mycobacterium leprae),* leishmaniasis and acute paronychia. The viral infections can cause viral warts (human papilloma virus), varicella (chickenpox), herpes zoster (varicella-zoster), herpes simplex (herpesvirus hominis), molluscum contagiosum, orf, AIDS (acquired immunodeficiency syndrome, human immunodeficiency virus, HIV), herpangina, mucocutaneous lymph node syndrome (Kawasaki's disease), Gianotti-Crosti syndrome (hepatitis B virus), measles, rubella and erythema infectiosum. The fungal infections can cause ringworm, tinea pedis (athlete's foot), tinea unguis (nail infection), tinea hands, tinea groin, tinea trunk and limbs, tinea capitis (scalp), oral candidiasis, candida intertrigo, genital candidiasis, chronic paronychia, chronic mucocutaneous candidiasis, pityriasis versicolor, histoplasmosis, coccidioidomycosis, blastomycosis, sporotrichosis, actinomycosis and mycetoma (madura foot).

### (G) Other Tissues or Systems

These conditions and diseases include tremor or shaking, obesity, vision disorders of eyes, vocal dysfunctions, gum and periodontal diseases, hearing loss, sexual dysfunctions, desired augmentation of breast and penis, to control, reduce or lose appetite for food, and increased body strength. Aside from cataract and glaucoma, the vision disorders can be due to near-sightedness (myopia) and far-sightedness (hyperopia). Enhanced strength of extrinsic and intrinsic eye muscles, along with increased relaxation of eye nerves may help improve conditions of myopia and hyperopia.

Weakness and poor quality of the voice can be caused by larynx dysfuction. Relaxation of laryngeal nerves and enhanced laryngeal muscle may help improve the quality and the strength of the voice.

The preferred condition or disease to be treated according to embodiments of the present invention is selected from the group consisting of arthritis, cancer, immune, infections, inflammation, musculus, nerve, skin, vasculature and obesity.

The more preferred condition or disease to be treated is selected from the group consisting of obesity, tremor or shaking, arthritis, Alzheimer's disease, aging related skin changes, age spots, breast cancer, cellulitis, dermatitis, dermatoses, dry skin, eczema, itch, infections, inflammation, joint disorder, mottled skin, muscle disorder, pain, Parkinson's disease, photoaging, psoriasis, rosacea, stretch marks, varicose veins, viral infections, wrinkles, for skin lightening, to enhance muscle strength; to induce relaxation; to reduce blood pressure or hypertension; to control, reduce or lose appetite; and to reduce or prevent sweating or perspiration of underarm, crotch, palm, or other parts of the body.

The most preferred condition or disease to be treated is selected from the group consisting of tremor, arthritis, joint disorder, breast cancer, Alzheimer's disease, Parkinson's disease, psoriasis, aging related skin changes, age spots, wrinkles, cellulitis, eczema, itch, inflammation, mottled skin, rosacea, stretch marks, and for skin lightening.

### Physiological Functions, Pharmacological Actions and Therapeutic Effects.

When a substance is found to modulate or normalize certain physiological functions, the resulting pharmacological actions can provide broad therapeutic effects on related conditions, disorders, diseases, symptoms and syndromes; simply described as "related indications". Therefore, the related indications can be grouped into one single physiological function as follows.
**(1) Disturbed keratinization (DK).** Many skin disorders such as dry skin, ichthyosis, calluses, keratosis and acne (initiated by blackhead formation) are due to disturbed keratinization (disturbed or abnormal skin formation). When a substance is discovered to modulate or normalize keratinization, the substance is reasonably expected or predicted to improve those conditions or disorders which are caused by a common cause of disturbed keratinization.
   Therefore, disturbed keratinization covers, but is not limited to dry skin; dryness or looseness of skin, nail and hair; xerosis; ichthyosis; calluses; keratoses; acne; rosacea; blemished skin; dandruff; uneven skin tone; uneven and rough surface of skin; abnormal skin texture and pores; flakiness and redness; and to improve or make skin soft, smooth, fresh, balanced, and visibly clear.
**(2) Aging related changes of skin, nail and hair (AG).** Skin aging including wrinkles is due mainly to progressive degeneration of dermal components; namely, glycosaminoglycans (GAGs), collagen and elastic fibers in the dermis. When a substance is found to stimulate biosynthesis of new dermal components or to plump the skin by increasing the skin thickness, the substance is reasonably expected or predicted to improve fine lines, wrinkles, photoaging, and to provide younger-looking skin.
   Therefore, aging related skin changes covers, for example, fine lines; wrinkles; age spots; blotches; cellulite; elastosis; lentigine; mottled skin; photoaging and photodamage; stretch marks; thinning of skin, nail plate and hair; warts; wrinkles; breakdown, defective synthesis or repair of dermal components; abnormal or diminished synthesis of collagen, glycosaminoglycans, proteoglycans and elastin, as well as diminished levels of such components in the dermis; loss or reduction of skin, nail and hair resiliency, elasticity and recoilability; laxity; lack of skin, nail and hair lubricants and luster; fragility and splitting of nail and hair; yellowing skin; and dull and older-looking skin, nail and hair, even-toned and brighter; to increase skin fullness and plumpness.
**(3) Deranged immune disorders and Inflammation (DI).** The deranged or disturbed immune disorders can cause inflammation, pain, itch, swelling, edema, dermatitis, eczema, psoriasis, dermatoses, joint disorders, and arthritis. When a substance is found to modulate or normalize activities of immune cells by reducing inflammation, the substance is reasonably expected or predicted to improve the related indications or disorders.
   Therefore, the deranged immune disorders cover, for example, inflammatory disorders; inflammation, dermatitis, or eczema; psoriasis; dermatoses; painful joints; arthritis; infections; Type 1 diabetes; viral hepatitis; inflammatory bowel disease; allergic food reactions; nephritis; vasculitis; vitiligo; multiple sclerosis; HIV and AIDS.
**(4). Tumors and cancers (CA).** Most tumors and cancers are caused by unregulated proliferation of cells due to loss of normal controls, resulting in abnormal growth, lack of differentiation, local tissue invasion, and often, metastasis. When a substance is found to normalize the control of cell growth, the substance is reasonably expected or predicted to improve or eradicate most types of tumors and cancers including skin tumors and cancers, breast cancer, lung carcinoma, liver cancer, pancreatic cancer, colon cancers, and brain tumors.
   Therefore, tumors and cancers cover: adrenal cancer, anus cancer, brain tumor and cancer, bladder cancer, breast cancer, cervix cancer, colon cancers, endometrium cancer, esophagus cancer, Kaposi sarcoma, kidney cancer, larynx cancer, leukemia, lymphoma, lung cancer, liver cancer, oral cavity cancer, ovarian cancer, prostate cancer, pancreatic cancer, rectum cancer, skin cancer, stomach cancer, testis cancer, thyroid cancer, and uterine cancer.
**(5). Nerve disorders (ND).** Nervous system is very complex, initiating from the brain and controlling almost all the body functions. Only the dead cells or dead tissues such as nails, hair and stratum corneum do not contain nerve fibers. Loss or malfunction of nerve cells can result in various nerve disorders, symptoms and syndromes.

Therefore, nerve disorders cover: nervousness, dementia, Alzheimer's disease: progressive loss of memory, carpal tunnel syndrome, weakness, pain, tingling, numbness, burning in palm and fingers, encephalitis, headache, migraine, meningitis, neuralgia, peripheral neuropathy, sciatica, Parkinson's disease, tremor, amnesia, Bell's palsy, epilepsy, multiple sclerosis, paralysis and headache.

In view of the present disclosure, standard procedures can be performed to evaluate the effect of the administration of a composition to a subject, thus allowing a skilled artisan to determine the therapeutically effective amount of the compound.

The clinically observable beneficial effect can be a situation that, when a composition of the present invention is administered to a subject after symptoms to be treated are observable, the symptoms are prevented from further development or aggravation, or develop to a lesser degree than without administration of the specified composition according to embodiments of the present invention. The clinically observable beneficial effect can also be that, when a composition of the present invention is administered to a subject before symptoms to be treated are observable, the symptoms are prevented from occurring or subsequently occur to a lesser degree than without administration of the composition of the present invention.

In one embodiment, a therapeutically effective amount of the N-acylpeptide derivative will reduce a syndrome or a condition of discomfort of the subject to be treated by at least about 20%, for example, by at least about 30%, about 40%, about 50%, about 60%, about 70%, about 80%, about 90%, or about 100%.

In another embodiment, a therapeutically effective amount of the N-acylpeptide derivative will prevent a syndrome or a condition of discomfort of the subject to be treated, or reduce the probability of its onset by at least about 20%, for example, by at least about 30%, about 40%, about 50%, about 60%, about 70%, about 80%, about 90%, or about 100%.

Dosages and dosing frequency will be determined by a trained medical professional depending on the activity of the compounds used, the characteristics of the particular topical formulation, and the identity and severity of the dermatologic disorder to be treated or prevented.

### Administration Routes and General Preparations

Another general aspect of the present invention relates to a composition for systemic or topical administration to a subject, the composition comprising a therapeutically effective amount of an N-acylpeptide derivative according to an embodiment of the present invention and a pharmaceutically or cosmetically acceptable carrier. Compositions according to embodiments of the present invention can be formulated in any manner suited for topical or systemic administration to a subject.

Compositions comprising an N-acylpeptide derivative of the present invention can be administered to a subject in need by topical application, systemic or other routes. The topical application includes administration to skin, eye, mucous membranes of the conjunctiva, nasopharynx, oropharynx, vagina, urethra, rectum, and anus. The systemic administration includes oral (enteral) administration and parenteral injections. The parenteral injections include intravenous injection or infusion, intra-arterial injection, subcutaneous injection, intramuscular injection, and intra-articular injection. Other routes of administration include sublingual administration, under the tongue, from oral mucosa bypassing the portal circulation, and pulmonary adsorption by inhaling and absorbing through the respiratory tract.

For topical application, the composition comprising an N-acylpeptide derivative of the present invention can be formulated as a solution, gel, lotion, cream, oil-in-water emulsion, water-in-oil emulsion, ointment, shampoo, spray, stick, powder, mask, pad, mouth rinse or wash, vaginal gel or suppository, rectal gel or suppository, urethral gel or suppository or other form acceptable for use on skin, nail, hair, oral mucosa, vaginal or anal mucosa, mouth or gums. The concentration of an active ingredient can be about 0.001% to about 99.9% by weight or volume of the total composition, with a preferred concentration of about 0.01% to about 30%, and with a more preferred concentration of about 0.1% to about 10% by weight or by volume (solution composition) of the total composition.

A typical gel composition can be formulated by the addition of a gelling agent, such as chitosan, methyl cellulose, ethyl cellulose, polyvinyl alcohol, polyquaterniums, hydroxyethylcellulose, hydroxypropylcellulose, hydroxypropylmethylcellulose, carbomer or ammoniated glycyrrhizinate to a solution comprising an N-acylpeptide derivative of the present invention. The preferred concentration of the gelling agent may range from 0.1 to 4 percent by weight of the total composition. In the preparation of shampoo, the N-acylpeptide derivative can first be dissolved in water or propylene glycol, and the solution thus obtained can be mixed with a shampoo base. Concentrations of the N-acylpeptide derivative used in gel or shampoo form are the same as described above.

To prepare a solution composition, at least one N-acylpeptide derivative of the present invention is dissolved in a solution prepared from water, ethanol, propylene glycol, butylene glycol, or other topically acceptable solvent. To prepare a topical composition in another form, an N-acylpeptide derivative can be incorporated as a fine powder form without dissolving, or is first dissolved in water, ethanol, propylene glycol or other solvent, and the solution thus obtained is mixed with a topically acceptable base or vehicle including a gel, lotion, cream, oil-in-water emulsion, water-in-oil emulsion, ointment, shampoo, spray, stick, powder, mask, pads, mouth rinse or wash, vaginal gel or suppository, and rectal gel or suppository. Contemplated embodiments of the present invention include concentration ranges of 0.001% to 0.01%, 0.01% to 0.1%, 0.1% to 0.2%, 0.2% to 0.3%, 0.3% to 0.4%, 0.4% to 0.5%, 0.5% to 0.6%, 0.6% to 0.7%, 0.7% to 0.8%, 0.8% to 0.9%, 0.9% to 1%, 1% to 2%, 2% to 3%, 3% to 4%, 4% to 5%, 5% to 6%, 6% to 7%, 7% to 8%, 8% to 9%, 9% to 10%, 10% to 14%, 14% to 18%, 18% to 22%, 22% to 26%, 26% to 30%, 30% to 35%, 35% to 40%, 40% to 45%, 45% to 50%, 50% to 60%, 60% to 70%, 70% to 80%, 80% to 90%, and 90% to 99.9% by weight or volume of the total composition.

The choice of topically administrable composition will depend on several factors, including the nature of the symptoms to be treated or prevented, the physiochemical characteristics of the particular compound to be administered and of other excipients present, their stability in the formulation, available manufacturing equipment, and cost constraints.

For systemic use or other routes of administration, an N-acylpeptide derivative of the present invention can be formulated for oral administration, parenteral injections or other routes including but not limited to oral mucosa, under the tongue administration with or without pharmaceutically acceptable vehicle or carrier.

In oral preparations, an N-acylpeptide derivative of the present invention is formulated in powder, tablet form, gelatin capsules with or without mixing with gelatin powder, or in other forms including a liquid or suspension form. Each tablet, capsule or unit dosage contains about 0.01 mg to about 100 mg, preferably about 0.1 mg to about 50 mg, and more preferably about 1 mg to about 25 mg of the N-acylpeptide derivative. As an illustrative example, 1 mg of N-acylpeptide derivative powder can be placed under the tongue without swallowing for a short time to achieve systemic administration. The daily dosage for a subject can vary, however in general is about 0.001 mg/kg to about 10 mg/kg, preferably about 0.01 mg to about 5 mg/kg, and more preferably about 0.1 mg to about 2 mg/kg body weight of the subj ect.

For parenteral injections, an N-acylpeptide derivative is prepared in a solution or suspension under sterilized conditions in concentration from about 0.01% to about 10%, preferably about 0.1% to about 5%, more preferably about 0.2% to about 2% weight by volume in water, propylene glycol, glycerol, polyethylene glycol, a mixture thereof, or in other vehicle or carrier. The other vehicle or carrier includes peanut oil, soybean oil, mineral oil, sesame oil, and the like. A thickener can optionally be added into an injection composition to increase the viscosity, so that the composition has a comparable viscosity with the body fluid in the knee joints or other joints. As an illustration, but not limitation, the thickener can be selected from the group consisting of carboxymethylcellulose, sodium carboxymethylcellulose, casein, cellulose, gelatin, sodium hyaluronate, methylcellulose, PEG 200, PEG 300, PEG 400, PEG 600, PEG 3350, PEG 4000, polyglactin, polylactide, polypropylene glycol, polyvinyl alcohol, protamine sulfate, povidone, starch, captisol, dextran, dextrose, fructose, albumin, and lactose.

In another embodiment, the composition can further comprise an additional cosmetic, pharmaceutical, or other agent to achieve synergetic or synergistic effects. To prepare a topical combination composition, a cosmetic, pharmaceutical or other agent is incorporated into any one of the above compositions by dissolving or mixing the agent into the formulation. Other forms of compositions for delivery of the N-acylpeptide derivative of the present invention are readily recognized by those skilled in the art.

A composition comprising the N-acylpeptide derivative can be taken orally one to three times, preferably twice daily, for prevention or treatment of disorders and diseases associated with tumors, cancers, immune, nervous, vascular, musculoskeletal, cutaneous, other tissues or systems. The oral administration may continue until the symptom or disease has been eradicated or substantially improved. The symptoms or disorders include, for example, pains, pruritus, tremor, inflammation, erythema, dermatitis, acne, eczema, dementia, Alzheimer's disease, joint pain or swelling, and arthritis.

The N-acylpeptide derivatives of the present invention are therapeutically effective to alleviate or improve conditions, disorders, diseases, symptoms or syndromes associated with immune, nervous, vascular, musculoskeletal, cutaneous, other tissues or systems, and for regulation and treatment of abnormal cell growth including tumors and cancers. The composition containing an N-acylpeptide derivative of the present invention can be administered alone or in combination with another active agent. The composition and the other active agent can be administered simultaneously or sequentially.

Other forms of compositions for delivery of the compound of the present invention are readily blended, prepared or formulated by those skilled in the art.

A composition comprising an N-acylpeptide derivative of the present invention is administered to a subject in various means that are acceptable for the conditions to be treated.

In one embodiment, the composition was topically applied to the skin. For example, a solution or cream containing 0.1% to 1 % by weight of an N-acylpeptide derivative, such as N-Ac-L-Tyr-L-Tyr-L-Tyr-NH2 or N-Ac-L-Tyr-L-Tyr-L-Tyr-OEt was topically applied to an involved skin once or twice daily for several weeks or until a desired therapeutic effect had been achieved.

The composition can also be administered systemically or by other routes, such as via oral administration or parenteral injection. For example, N-Ac-L-Tyr-L-Tyr-L-Tyr-NH2 0.2% (w/v) in water, 1 ml (2 mg) can be injected intra-articularily into a knee of a subject to relieve the arthritis pain and inflammation.

The composition can be administered alone or optionally in combination with another active ingredient. For example, a corticosteroid, hydrocortisone-17-valerate 0.2% (w/v) can be incorporated into a topical composition containing 0.5% (w/v) N-Ac-L-Tyr-L-Tyr-L-Tyr-NH2 to rapidly improve chronic eczema lesions. The composition and the other active ingredient can be administered topically, systemically, simultaneously or sequentially. Under such cooperative actions, the N-acylpeptide derivative and other active ingredient can mutually provide synergetic, synergistic, or enhancing effects for the intended treatment.

For synergetic, synergistic, additive, enhancing, or other mutually cooperative beneficial effects, a cosmetic, pharmaceutical, or other agent can be incorporated into the composition of the present invention or administered independently at the same time or different time. These agents include but are not limited to hydroxyacids, ketoacids and related compounds; phenyl alpha acyloxyalkanoic acids and derivatives; N-acyl-aldosamines, N-acylamino acids and related N-acyl compounds; local analgesics and anesthetics; anti-acne agents; anti-bacterial agents; anti-yeast agents; anti-fungal agents; anti-viral agents; anti-infective agents; anti-dandruff agents; anti-dermatitis agents; anti-eczema agents; anti-histamine agents; anti-pruritic agents; anti-emetics; anti-motion sickness agents; anti-inflammatory agents; anti-hyperkeratotic agents; antiperspirants; anti-psoriatic agents; anti-rosacea agents; anti-seborrheic agents; hair conditioners and hair treatment agents; anti-aging and anti-wrinkle agents; anti-anxiety agents; anti-convulsant agents; anti-depressant agents; antineoplastic agents; sunblock and sunscreen agents; skin lightening agents; depigmenting agents; astringents; cleansing agents; corn, callus and wart removing agents; skin plumping agents; skin volumizing agents; skin firming agents; matrix metalloproteinase (MMP) inhibitors; topical cardiovascular agents; wound-healing agents; gum disease or oral care agents; amino acids; tripeptides; oligopeptides; polypeptides; carbohydrates; aminocarbohydrates; vitamins; corticosteroids; tanning agents; hormones; retinoids; peroxides; peracids; superoxides, ozonides, persulfates, and active agents.

The above agents include, but are not limited to, the following: abacavir, abciximab, abelcet, acamprosate, acarbose, acebutolol, acetaminophen, acetaminosalol, acetazolamide, acetic acid, acetic peracid, acetic peroxide, acetohydroxamic acid, acetylcysteine, acetylsalicylic acid, N-acylglutathione esters, acitretin, aclovate, acrivastine, acthrel, actidose, actigall, actiq, acyclovir, adalimumab, adapalene, adefovir dipivoxil, adenosine, agalsidase, albendazole, albumin, albuterol, alclometasone dipropionate, aldesleukin, alefacept, alemtuzumab, alendronate, alfuzosin, alitretinoin, allantoin, allium, allopurinol, alloxanthine, almotriptan, alosetron, alpha tocopheral, alphal-proteinase, alprazolam, alprenolol, alprostadil, alteplase, altretamine, aluminum acetate, aluminum chloride, aluminum chlorohydroxide, aluminum hydroxide, amantadine, amifostine, amiloride, aminacrine, amino acid, aminobenzoate, p-aminobenzoic acid, aminocaproic acid, aminohippurate, aminolevulinic acid, aminosalicylic acid, amiodarone, amitriptyline, amlodipine, amocarzine, amodiaquin, amorolfine, amoxapine, amoxicillin, amphetamine, amphotericin, ampicillin, amprenavir, anagrelide, anakinra, anastrozole, anisindione, anthralin, antihemophilic, antithrombin, anti-thymocyte, antivenin, apomorphine, aprepitant, aprotinin, arbutin, argatroban, aripiprazole, ascorbic acid, ascorbyl palmitate, aspirin, atazanavir, atenolol, atomoxetine, atorvastatin, atovaquone, atropine, azathioprine, azelaic acid, azelastine, azithromycin, baclofen, bacitracin, balsalazide, balsam, basiliximab, beclomethasone dipropionate, bemegride, benazepril, bendroflumethiazide, benzocaine, benzoic acid, benzonatate, benzophenone, benzoyl peroxide, benztropine, bepridil, beta carotene, betamethasone dipropionate, betamethasone valerate, betaxolol, bethanechol, bevacizumab, bexarotene, bicalutamide, bimatoprost, bioflavonoids, biotin, biperiden, bisacodyl, bisoprolol, bivalirudin, bortezomib, bosentan, botulinum, brimonidine, brinzolamide, bromocriptine, brompheniramine, budesonide, bumetanide, bupivacaine, buprenorphine, bupropion, burimamide, buspirone, busulfan, butabarbital, butalbital, butenafine, butoconazole, butorphanol, butyl aminobenzoate, cabergoline, caffeic acid, caffeine, calcipotriene, calcitonin-salmon, calcitriol, calcium peroxide, calfactant, camellia sinensis, camphor, candesartan cilexetil, capecitabine, capreomycin, capsaicin, captopril, carbamazepine, carbamide peroxide, carbidopa, carbinoxamine, cefditoren pivoxil, cefepime, cefpodoxime proxetil, celecoxib, cetirizine, cevimeline, chitosan, chlordiazepoxide, chlorhexidine, chloroquine, chlorothiazide, chloroxylenol, chlorpheniramine, chlorpromazine, chlorpropamide, ciclopirox, cilostazol, cimetidine, cinacalcet, ciprofloxacin, citalopram, citric acid, cladribine, clarithromycin, clemastine, clindamycin, clioquinol, clobetasol propionate, clocortolone pivalate, clomiphene, clonidine, clopidogrel, clotrimazole, clozapine, coal tar, coal tar extracts (LCD), codeine, cromolyn, crotamiton, cyclizine, cyclobenzaprine, cycloserine, cytarabine, dacarbazine, dalfopristin, dapsone, daptomycin, daunorubicin, deferoxamine, dehydroepiandrosterone, delavirdine, desipramine, desloratadine, desmopressin, desoximetasone, dexamethasone, dexmedetomidine, dexmethylphenidate, dexrazoxane, dextroamphetamine, diazepam, diclofenac, dicyclomine, didanosine, dihydrocodeine, dihydromorphine, diltiazem, 6,8-dimercaptooctanoic acid (dihydrolipoic acid), diphenhydramine, diphenoxylate, dipyridamole, disopyramide, dobutamine, dofetilide, dolasetron, donepezil, dopa esters, dopamide, dopamine, dorzolamide, doxepin, doxorubicin, doxycycline, doxylamine, doxypin, duloxetine, dyclonine, econazole, efalizumab, eflornithine, eletriptan, emtricitabine, enalapril, ephedrine, epinephrine, epinine, epirubicin, eptifibatide, ergotamine, erythromycin, escitalopram, esmolol, esomeprazole, estazolam, estradiol, etanercept, ethacrynic acid, ethinyl estradiol, etidocaine, etomidate, famciclovir, famotidine, felodipine, fentanyl, ferulic acid, fexofenadine, flecainide, fluconazole, flucytosine, fluocinolone acetonide, fluocinonide, 5-fluorouracil, fluoxetine, fluphenazine, flurazepam, fluticasone propionate, fluvoxamine, formoterol, furosemide, galactarolactone, galactonic acid, galactonolactone, galantamine, gatifloxacin, gefitinib, gemcitabine, gemifloxacin, glucarolactone, gluconic acid, gluconolactone, glucuronic acid, glucuronolactone, glutathione, glycolic acid, griseofulvin, guaifenesin, guanethidine, N-guanylhistamine, haloperidol, haloprogin, hexylresorcinol, homatropine, homosalate, hormone, hydralazine, hydrochlorothiazide, hydrocortisone, hydrocortisone 21-acetate, hydrocortisone 17-butyrate, hydrocortisone 17-valerate, hydrogen peroxide, hydromorphone, hydroquinone, hydroquinone monoether, hydroxyzine, hyoscyamine, hypoxanthine, ibuprofen, ichthammol, idarubicin, imatinib, imipramine, imiquimod, indinavir, indomethacin, infliximab, irbesartan, irinotecan, isoetharine, isoproterenol, itraconazole, kanamycin, ketamine, ketanserin, ketoconazole, ketoprofen, ketotifen, kojic acid, labetalol, lactic acid, lactobionic acid, lamivudine, lamotrigine, lansoprazole, letrozole, leuprolide, levalbuterol, levofloxacin, lidocaine, linezolid, lobeline, loratadine, loperamide, losartan, loxapine, lysergic diethylamide, mafenide, malic acid, maltobionic acid, mandelic acid, maprotiline, mebendazole, mecamylamine, meclizine, meclocycline, memantine, menthol, meperidine, mepivacaine, mequinol, mercaptopurine, mescaline, metanephrine, metaproterenol, metaraminol, metformin, methadone, methamphetamine, methotrexate, methoxamine, methyldopa esters, methyldopamide, 3,4-methylenedioxymethamphetamine, methyllactic acid, methyl nicotinate, methylphenidate, methyl salicylate, metiamide, metolazone, metoprolol, metronidazole, mexiletine, miconazole, midazolam, midodrine, miglustat, minocycline, minoxidil, mirtazapine, mitoxantrone, moexiprilat, molindone, monobenzone, morphine, moxifloxacin, moxonidine, mupirocin, nadolol, naftifine, nalbuphine, nalmefene, naloxone, naproxen, nefazodone, nelfinavir, neomycin, nevirapine, niacin, niacinamide, nicardipine, nicotine, nifedipine, nimodipine, nisoldipine, nitrofurantoin, nizatidine, norepinephrine, nystatin, octopamine, octreotide, octyl methoxycinnamate, octyl salicylate, ofloxacin, olanzapine, olmesartan medoxomil, olopatadine, omeprazole, ondansetron, oxiconazole, oxotremorine, oxybenzone, oxybutynin, oxycodone, oxymetazoline, padimate O, palonosetron, pantothenic acid, pantoyl lactone, paroxetine, pemoline, penciclovir, penicillamine, penicillins, pentazocine, pentobarbital, pentostatin, pentoxifylline, peptide, perazine, pergolide, perindopril, permethrin, phencyclidine, phenelzine, pheniramine, phenmetrazine, phenobarbital, phenol, phenoxybenzamine, phentolamine, phenylephrine, phenylpropanolamine, phenytoin, physostigmine, pilocarpine, pimecrolimus, pimozide, pindolol, pioglitazone, pipamazine, piperonyl butoxide, pirenzepine, podofilox, podophyllin, potassium peroxide, povidone iodine, pramipexole, pramoxine, prazosin, prednisone, prenalterol, prilocaine, procainamide, procaine, procarbazine, promazine, promethazine, promethazine propionate, propafenone, propoxyphene, propranolol, propylthiouracil, protein, protriptyline, pseudoephedrine, pyrethrin, pyrilamine, pyrimethamine, quetiapine, quinapril, quinethazone, quinidine, quinupristin, rabeprazole, reserpine, rcsorcinol, retinal, 13-cis-retinoic acid, retinoic acid, retinol, retinyl acetate, retinyl palmitate, ribavirin, ribonic acid, ribonolactone, rifampin, rifapentine, rifaximin, riluzole, rimantadine, risedronic acid, risperidone, ritodrine, rivastigmine, rizatriptan, ropinirole, ropivacaine, salicylamide, salicylic acid, salmeterol, scopolamine, selegiline, selenium sulfide, serotonin, sertaconazole, sertindole, sertraline, shale tar, sibutramine, sildenafil, sotalol, streptomycin, strychnine, sulconazole, sulfacetamide, sulfabenz, sulfabenzamide, sulfabromomethazine, sulfacetamide (sodium sulfacetamide), sulfachlorpyridazine, sulfacytine, sulfadiazine, sulfadimethoxine, sulfadoxine, sulfaguanole, sulfalene, sulfamethizole, sulfamethoxazole, sulfanilamide, sulfapyrazine, sulfapyridine, sulfasalazine, sulfasomizole, sulfathiazole, sulfisoxazole, sulfur, tacrolimus, tadalafil, tamsulosin, tartaric acid, tazarotene, tegaserol, telithromycin, telmisartan, temozolomide, tenofovir disoproxil, terazosin, terbinafine, terbutaline, terconazole, terfenadine, tetracaine, tetracycline, tetrahydrozoline, thalidomide, theobromine, theophylline, thiabendazole, thiethylperazine, thioctic acid (lipoic acid), thioridazine, thiothixene, thymol, tiagabine, timolol, tinidazole, tioconazole, tirofiban, tizanidine, tobramycin, tocainide, tolazoline, tolbutamide, tolnaftate, tolterodine, tramadol, tranylcypromine, trazodone, triamcinolone acetonide, triamcinolone diacetate, triamcinolone hexacetonide, triamterene, triazolam, triclosan, triflupromazine, trimethoprim, trimipramine, tripelennamine, triprolidine, tromethamine, tropic acid, tyramine, undecylenic acid, urea, urocanic acid, ursodiol, valacyclovir, vardenafil, venlafaxine, verapamil, vitamin, vitamin E acetate, voriconazole, warfarin, wood tar, xanthine, zafirlukast, zaleplon, zinc pyrithione, ziprasidone, zolmitriptan and zolpidem.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

### Example 1

In one of the studies related to skin changes associated with aging, skin thickness was measured by micrometer calipers as follows.

The skin was grasped with a 2 X 6 cm metal hinge; the internal faces of which were coated with emery cloth to prevent slippage, and manually squeezed to threshold subject discomfort. Combined thickness of two whole-skin layers including thickness of the two hinge leaves was measured with micrometer calipers. Thickness of the two hinge leaves was subtracted to determine the actual thickness of two whole-skin layers. Triplicate measurements on treated sites were done and an average number was used for calculation of the skin thickness.

In other studies, test sites of skin 17 mm in diameter were used, the circular sites were marked with permanent ink. Intervening control sites were also 17 mm in diameter. Thickness of skin of all sites was measured directly by means of an electronic digital caliper. In this instance the jaws of the caliper were opened to 17 mm, applied with pressure to the skin sites and then closed to firm tightness. Thickness of skin was then read off the screen of the calipers. Measurements of all sites were made in triplicates.

### Example 2

Solution compositions containing an N-acylpeptide derivative of the present invention were formulated as follows.

N-Ac-L-Tyr-L-Tyr-L-Tyr-NH₂, 0.3 g was dissolved in 99.7 ml solution prepared from water 40 parts, ethanol 40 parts and propylene glycol 20 parts by volume (hereinafter referred to as WEP442). Other proportion such as WEP721 refers to water 70 parts, ethanol 20 parts and propylene glycol 10 parts by volume. The clear solution thus prepared had pH 5.9 and contained N-Ac-L-Tyr-L-Tyr-L-Tyr-NH₂, 0.3% (w/v) in WEP442. Under the same conditions, N-Ac-L-Tyr-L-Tyr-L-Tyr-NH₂, 0.1 to 1 % (w/v) in WEP442 was readily formulated. EP73 indicates ethanol 70 parts and propylene glycol 30 parts by volume.

N-Ac-L-Tyr-L-Tyr-L-Tyr-OEt, 0.4 g was dissolved in 99.6 ml solution prepared from ethanol 80 parts and propylene glycol 20 parts by volume (hereinafter referred to as EP82). The clear solution thus prepared contained N-Ac-L-Tyr-L-Tyr-L-Tyr-OEt, 0.4% (w/v) in EP82.

Under the same procedures, the following solution compositions were prepared:
N-Ac-L-Tyr-L-Tyr-L-Tyr-OEt: 0.4-1% (w/v) in WEP442, pH 5.6; 1-2% (w/v) in EP73;
N-Ac-L-Ser-L-Ser-L-Ser-NH₂: 0.3% (w/v) in WEP442, pH 2.9; 0.5% (w/v) in WEP721;
N-Ac-L-Arg-L-Arg-L-Arg-NH₂: 0.3% (w/v) in WEP 442, pH 3.0; 0.5% (w/v) in WEP721;
N-Ac-L-Tyr-L-Tyr-L-Tyr-OH: 0.2-1% (w/v) in WEP442, pH 4.8; 0.5-1% (w/v) in WEP721;
N-Ac-L-Val-L-Val-L-Ala-NH₂: 0.2% (w/v) in WEP442, pH 4.9;
N-Ac-L-Val-L-Tyr-L-Tyr-NH₂: 0.2% (w/v) in WEP 442, pH 4.0;
N-Ac-L-Tyr-L-Tyr-L-Tyr-NH₂: 0.5% (w/v) in WEP 721, pH 5.2;
N-Ac-L-Val-L-Ala-L-Tyr-NH₂: 0.3% (w/v) in WEP 442, pH 5.5;
N-Ac-L-Ala-L-Leu-L-Lys-L-His-L-Arg-NH₂ (SEQ ID NO: 51): 0.2% (w/v) in WEP442, pH 3.4; 0.5%(w/v) in WEP721;
N-Pr-L-Ala-L-Leu-L-Lys-L-His-1-Arg-NH₂ (SEQ ID NO: 58): 0.2% (w/v) in WEP442, pH 3.8; 0.5%(w/v) in WEP721; and
N-Bz-L-Ala-L-Leu-L-Lys-L-His-L-Arg-NH₂ (SEQ ID NO: 65): 0.2% (w/v) in WEP442, pH 6.0; 0.5%(w/v) in WEP721.

The solution compositions containing N-acylpeptide derivatives of the present invention are therapeutically effective for topical treatment of disorders, diseases, symptoms or syndromes associated with tumors, cancers, immune, nervous, vascular, musculoskeletal or cutaneous system, or other tissues or systems.

### Example 3

Creams or oil-in-water emulsions containing an acylpeptide derivative were formulated as follows:

N-Ac-L-Tyr-L-Tyr-L-Tyr-OEt, 0.2 g was dissolved in warm propylene glycol 20 ml and oleyl lactate 10 ml, and the solution thus obtained was mixed with a cream or oil-in-water emulsion 69.8 g. The composition thus formulated contained N-Ac-L-Tyr-L-Tyr-L-Tyr-OEt, 0.2% (w/w) in cream or oil-in-water emulsion.

N-Ac-L-Tyr-L-Tyr-L-Tyr-NH₂, 0.2 g was dissolved in warm propylene glycol 15 ml and water 15 ml, and the solution thus obtained was mixed with a cream or oil-in-water emulsion 69.8 g. The composition thus formulated contained N-Ac-L-Tyr-L-Tyr-L-Tyr-NH₂, 0.2% (w/w) in cream or oil-in-water emulsion.

N-Ac-L-Arg-L-Arg-L-Arg-NH₂, 0.2 g was dissolved in warm propylene glycol 6 ml and water 24 ml, and the solution thus obtained was mixed with a cream or oil-in-water emulsion 69.8 g. The composition thus formulated contained N-Ac-L-Arg-L-Arg-L-Arg-NH₂, 0.2% (w/w) in cream or oil-in-water emulsion.

Under the same procedures, the following cream or oil-in-water compositions were formulated:
N-Ac-L-Ser-L-Ser-L-Ser-NH₂: 0.2% (w/w) in cream or oil-in-water emulsion;
N-Ac-L-Tyr-L-Tyr-L-Tyr-OEt: 1% (w/w) in cream or oil-in-water emulsion;
N-Ac-L-Ala-L-Leu-L-Lys-L-His-L-Arg-NH₂ (SEQ ID NO: 51): 0.5% (w/w) in cream or oil-in-water emulsion; and
N-Pr-L-Ala-L-Leu-L-Lys-L-His-L-Arg-NH₂ (SEQ ID NO: 58): 0.5%(w/w) in cream or oil-in-water emulsion.

The cream or oil-in-water emulsion compositions containing N-acylpeptide derivatives of the present invention can be therapeutically effective for topical treatment of disorders, diseases, symptoms or syndromes associated with tumors, cancers, immune, nervous, vascular, musculoskeletal or cutaneous system, or other tissues or systems.

### Example 4

A typical solution composition containing N-acylpeptide derivative of the present invention for systemic injection administration was prepared as follows.

A composition can be prepared with or without a thickening agent, such as methyl cellulose. Methyl cellulose 1% (w/v) in water solution was prepared by adding methyl cellulose 1 g in water 90 ml, and the mixture was gently homogenized. More water was added to make the final volume of 100 ml. The vehicle composition thus prepared contained 1% (w/v) methyl cellulose as a thickener in injection solution.

N-Ac-L-Tyr-L-Tyr-L-Tyr-OH, 100 mg was dissolved in water, 10 ml, and the solution thus obtained in an injection bottle was sterilized for 30 minutes in boiling water bath. The injection composition thus prepared contained N-Ac-L-Tyr-L-Tyr-L-Tyr-OH, 1% (w/v) in water.

N-Ac-L-Tyr-L-Tyr-L-Tyr-OEt, 20 mg was dissolved in water, 10 ml, and the solution thus obtained in an injection bottle was sterilized for 30 minutes in boiling water bath. The injection composition thus prepared contained N-Ac-L-Tyr-L-Tyr-L-Tyr-OEt, 0.2% (w/v) in water. Under the same procedure, the injection composition containing N-Ac-L-Tyr-L-Tyr-L-Tyr-OEt, 0.2% (w/v) and methyl cellulose 1% (w/v) in water was prepared by dissolving N-Ac-L-Tyr-L-Tyr-L-Tyr-OEt, 20 mg in 10 ml water containing 1% (w/v) methyl cellulose.

N-Ac-L-Ala-L-Leu-L-Lys-L-His-L-Arg-NH₂ (SEQ ID NO: 51), 40 mg was dissolved in 2 ml water containing 1% methyl cellulose as prepared above in an injection bottle, and the vial was sterilized at 100°C for 30 minutes. The solution compositions thus obtained contained 2% (w/v) or 20 mg/ml N-Ac-L-Ala-L-Leu-L-Lys-L-His-L-Arg-NH₂ (SEQ ID NO: 51) which was suitable for intra-articular, intralesional, or subcutaneous injection, or other systemic administration.

Under the same procedures, the following solution compositions for injection were prepared:
N-Ac-L-Ala-L-Leu-L-Lys-L-His-L-Arg-NH2 (SEQ ID NO: 51), 1% (w/v) in water;
N-Bz-L-Ala-L-Leu-L-Lys-L-His-L-Arg-NH2 (SEQ ID NO: 65), 1% (w/v) in water; and
N-Ac-(EEASPEAVAGVGFESK)-NH2 (SEQ ID NO: 126) 0.4% (w/v) or 4 mg/ml in water.

The solution compositions containing N-acylpeptide derivatives of the present invention can be therapeutically effective for systemic treatment of disorders, diseases, symptoms or syndromes associated with tumors, cancers, immune, nervous, vascular, musculoskeletal or cutaneous system, or other tissues or systems.

### Example 5

A typical in vitro screen for anti-tumor or anti-cancer effects was carried out as follows.

An aliquot of 2,000 MB231 breast cancer cells (breast carcinoma cells) in 100 µl DMEM complete media (Sigma Chemical Co.) was plated in a 96 well plate containing at a concentration of 20 µg/ml a test substance or control water. To measure proliferation of the cancer cells, an aliquot of 20 µl of MTS reagent (Promega Co.) was added to each well and the cells were incubated at 37°C for a total of three days. The cells rapidly metabolized MTS reagent and the metabolized MTS reagent was measured at 490 nm at the end of days 1, 2 and 3. The reading was proportional to the number of cancer cells. The decrease in absorbance at each time point indicated fewer cancer cells. At the end of day 3, the decrease in absorbance with respect to the control indicated the inhibition by the test substance.

Using the above procedure, N-Ac-(EEASPEAVAGVGBESK)-NH2 (SEQ ID NO: 145) at the concentration, 20 µg/ml was found to have 9% inhibition of breast cancer cells at the end of three day incubation.

The following N-acylpeptide derivatives of the present invention can also inhibit the growth of breast cancer cells.
N-Ac-L-Arg-L-Arg-L-Arg-NH₂, N-Ac-L-Tyr-L-Tyr-L-Tyr-NH₂,
N-Ac-L-Val-L-Val-L-Ala-NH₂, N-Ac-L-Val-L-Tyr-L-Tyr-NH₂,
N-Ac-(EASPEAVAGVGFESK)-NH₂ (SEQ ID NO: 105), N-Ac-(EEASPEAVAGVGFESK)-NH₂ (SEQ ID NO: 126), and N-Ac-(CKKEEASPEAVAGVGFESK)-NH₂ (SEQ ID NO: 149).

Thus, N-acypeptide derivatives of the present invention can be used for treating breast cancers.

### Example 6 (*: compositions of the invention)

A typical skin plump or skin thickness test was carried out as follows.

A female subject, age 42, selected 5 skin test spots, each with 17 mm in diameter on her distal extensor left forearm, marked A, B, C, D, and E. The subject topically applied three times daily to test skin spots:
A. N-Ac-L-Arg-L-Arg-L-Arg-NH₂, 0.5%(w/v) in WEP721
B. N-Ac-L-Tyr-L-Tyr-L-Tyr-NH₂, 0.5%(w/v) in WEP721 *
C. N-Ac-L-Tyr-L-Tyr-L-Tyr-OH 0.5%(w/v) in WEP721 *
D. N-Ac-L-Ala-L-Leu-L-Lys-L-His-L-Arg-NH₂ (SEQ ID NO: 51), 0.5%(w/v) in WEP721
E. Vehicle control, WEP721

The topical applications were continued for 7 days, and the skin thickness was measured by the electronic digital caliper as described in Example 1. At the end of 7 days, while the skin in the vehicle control spot E was still loose, relatively thin and wrinkled, the skins in spots A, B, C and D were palpably raised, smooth, less wrinkled and moderately increased in skin thickness. While there was no change in skin thickness of the control spot, the treated skins in spots A, B, C and D had approximately 5-10% increase in skin thickness as measured by the electronic micrometer caliper.

This result indicates that N-acylpeptide derivatives of the present invention can be used for topical treatment of disturbed keratinization and aging related changes of skin, nail and hair including fine lines, wrinkles, photoaging, age spots, blotches, mottled skin, stretch marks and for younger-looking skin and skin lightening.

### Example 7 (*: compositions of the invention)

A female subject, age 51, selected 5 skin test spots, each with 17 mm in diameter on her distal extensor left forearm, marked A, B, C, D, and E. The subject topically applied three times daily to test skin spots:
A N-Ac-L-Arg-L-Arg-L-Arg-NH₂, 0.5%(w/v) in WEP721
B. N-Ac-L-Tyr-L-Tyr-L-Tyr-NH₂, 0.5%(w/v) in WEP721 *
C. N-Ac-L-Tyr-L-Tyr-L-Tyr-OH 0.5%(w/v) in WEP721 *
D. N-Ac-L-Ala-L-Leu-L-Lys-L-His-L-Arg-NH₂ (SEQ ID NO: 51), 0.5%(w/v) in WEP721
E. Vehicle control, WEP721

The topical applications were continued for 7 days, and the skin thickness was measured by the electronic digital caliper as described in Example 1. At the end of 7 days, while the skin in the vehicle control spot E was still loose, relatively thin and wrinkled, the skins in spots A, B, C and D were palpably raised, smooth, less wrinkled and moderately increased in skin thickness. While there was no change in skin thickness of the control spot, the treated skins in spots A, B, C and D had approximately 5-10% increase in skin thickness as measured by the electronic micrometer caliper.

This result indicates that N-acylpeptide derivatives of the present invention can be used for topical treatment of disturbed keratinization and aging related changes of skin, nail and hair including fine lines, wrinkles, photoaging, age spots, blotches, mottled skin, stretch marks, and for younger-looking skin and skin lightening.

### Example 8

A female subject, age 74, had severe photodamage on her skins of both arms. The subject topically applied three times daily N-Ac-L-Tyr-L-Tyr-L-Tyr-NH₂, 0.5%(w/v) in WEP721to the skin over right wrist distal, and vehicle control WEP721 to left wrist distal for 7 days. At the end of 7 days, while the skin over the left wrist distal of the control was still loose, relatively thin and wrinkled, the skins over the right wrist distal was palpably raised, smooth, less wrinkled and moderately increased in skin thickness. While there was no change in skin thickness of the control skin over the left wrist distral, the skin of the treated right wrist distal had approximately 5-10% increase in skin thickness as measured by the electronic micrometer caliper. This result indicates that N-acylpeptide derivatives of the present invention can be used - for topical treatment of disturbed keratinization and aging related changes of skin, nail and hair including fine lines, wrinkles, photoaging, age spots, blotches, mottled skin, stretch marks, and for younger-looking skin and skin lightening.

### Example 9 (*: compositions of the invention)

A male subject, age 90, having severe photodamage of the skin in both forearms selected 5 skin test spots on his forearms, marked A, B, C, D, and E. The subject topically applied four times daily to test skin spots for 10 days.
A. N-Ac-L-Tyr-L-Tyr-L-Tyr-OEt, 0.5%(w/v) in WEP442 *
B. N-Ac-L-Tyr-L-Tyr-L-Tyr-OH, 0.5%(w/v) in WEP721 *
C. N-Pr-L-Ala-L-Leu-L-Lys-L-His-L-Arg-NH₂ (SEQ ID NO: 58), 0.5%(w/v) in WEP721
D. N-Bz-L-Ala-L-Leu-L-Lys-L-His-L-Arg-NH₂ (SEQ ID NO: 65), 0.5%(w/v) in WEP721
E. Vehicle control, WEP721

At the end of 10 days, while the skin in the vehicle control spot E was still loose, relatively thin and wrinkled, the skins in spots A, B, C and D were palpably raised, smooth, less wrinkled and increased in skin thickness. While there was no change in skin thickness of the control spot, the treated skins in spots B, C and D had approximately 10-20% increase in skin thickness as measured by the electronic micrometer caliper. The treated skin in spot A had approximately 20-30% increase in skin thickness as measured by the electronic micrometer caliper.

The above results indicate that N-acylpeptide derivatives of the present invention can be used for topical treatment of disturbed keratinization and aging related changes of skin, nail and hair including fine lines, winkles, photoaging, age spots, blotches, mottled skin, stretch marks, and for younger-looking skin and skin lightening.

### Example 10

A male subject, age 80, having chronic inflammation, erythema, eczema with thick scales and itch on his legs for more than 10 years, failed to respond with conventional treatments including topical corticosteroids. The involved skin was divided into two lesions for testing. The subject topically applied twice daily to one lesion, N-Ac-L-Tyr-L-Tyr-L-Tyr-NH₂ 0.3% (w/v) in WEP442, pH 5.9 solution composition, prepared according to Example 2, and to second lesion, N-Ac-L-Tyr-L-Tyr-L-Tyr-OEt 0.5% (w/v) in WEP 442, pH 6.0, prepared according to Example 2. The itch stopped within a few minutes, and the thick scales of both lesions started to disappear in the next few days. At the end of 2 weeks, the erythema and thick scales of both lesions disappeared almost completely and the treated skin sites were smooth, soft, even-toned, brighter and more elastic when the skin was stretched. The treated skin appeared much lighter in skin color as compared to the surrounding untreated skin site. The treated skin had 90-95% improvement as judged by clinical evaluation.

The result shows that N-acyltripeptide derivatives of the present invention can be used for providing therapeutic effects for topical treatment of symptoms or syndromes associated with nerve disorders, disturbed keratinization, aging skin, wrinkles, age spots, hyperpigmentation, inflammation, deranged immune system, and for skin lightening.

### Example 12

A male subject, age 41, having normal and regular eating habit, topically applied once N-Ac-L-Tyr-L-Tyr-L-Tyr-NH₂, 0.3% (w/v) in WEP442, pH 5.9 preprared according to Example 2 on his forehead one hour before the regular dinner time. After about 50-60 minutes of the topical application, the subject strated to feel loss of appetite for food. At the dinner time, the subject lost the desire to eat food, but managed to eat only half the amount of food he usually would consume.

The above result shows that N-acyltripeptide derivative of the present invention can be used for providing therapeutic effects for reducing or losing appetite for food, and for controlling body weight.

### Example 13

A male subject, age 36, having plaque psoriasis, topically applied twice daily N-Ac-L-Tyr-L-Tyr-L-Tyr-NH₂ 0.2% (w/w) in cream to plaque lesions for 3 weeks. The treated lesions started to improve after a few days of topical application. At the end of 3 weeks, the silvery scales disappeared almost completely, the intense erythema diminished substantially, and the thick skin became palpably thinner and also in appearance. The treated lesions had 40-50% improvement as judged by clinical evaluation.

The above result shows that N-acylpeptide derivative of the present invention can be used for topical treatment of psoriasis, inflammatory diseases and disorders associated with deranged immune system.

### Example 14 (*: compositions of the invention)

A typical preparation of gelatin capsules for systemic administration was carried out as follows.

N-Ac-L-Tyr-L-Tyr-L-Tyr-OEt powder 1g was mixed thoroughly with gelatin powder 14 g, and this powder mixture was filled into gelatin No. 3 capsules. Each capsule thus filled contained approximately 10 mg N-Ac-L-Tyr-L-Tyr-L-Tyr-OEt and 140 mg gelatin.

Under the same procedures, the following capsules can be readily prepared:
10 mg N-Ac-L-Tyr-L-Tyr-L-Tyr-OH and 140 mg gelatin *
10 mg N-Ac-L-Tyr-L-Tyr-L-Tyr-NH₂ and 140 mg gelatin *
30 mg N-Ac-L-Tyr-L-Tyr-L-Tyr-NH₂ and 120 mg gelatin *
50 mg N-Ac-L-Tyr-L-Tyr-L-Tyr-NH₂ and 100 mg gelatin *
10 mg N-Ac-L-Arg-L-Arg-L-Arg-NH₂ and 140 mg gelatin
10 mg N-Ac-L-Ser-L-Ser-L-Ser-NH₂ and 140 mg gelatin
10 mg N-Ac-L-Tyr-L-Tyr-L-Tyr-OH and 140 mg gelatin *
20 mg N-Ac-L-Val-L-Tyr-L-Tyr-NH₂ and 130 mg gelatin
20 mg N-Ac-L-Val-L-Val-L-Ala-NH₂ and 130 mg gelatin
5 mg N-Ac-L-Ala-L-Leu-L-Lys-L-His-L-Arg-NH₂ (SEQ ID NO: 51) and 145 mg gelatin
10 mg N-Pr-L-Ala-L-Leu-L-Lys-L-His-L-Arg-NH₂ (SEQ ID NO: 58) and 140 mg gelatin
10 mg N-Bz-L-Ala-L-Leu-L-Lys-L-His-L-Arg-NH₂ (SEQ ID NO: 65) and 140 mg gelatin
5 mg N-Ac-(EEASPEAVAGVGFESK)-NH₂ (SEQ ID NO: 126) and 145 mg gelatin
5 mg N-Ac-(EEASPEAVAGVGBESK)-NH₂ (SEQ ID NO: 145) and 145 mg gelatin

A subject can take orally the above capsules on the daily basis. The daily dosage for a subject can vary, however in general is about 0.001mg/kg to about 10 mg/kg, preferably about 0.01 mg to about 5 mg/kg, and more preferably about 0.1 mg to about 2 mg/kg body weight of the subject.

The capsule compositions containing N-acylpeptide derivatives of the present invention can be therapeutically effective for orally treatment of disorders, diseases, symptoms or syndromes associated with tumors, cancers, immune, nervous, vascular, musculoskeletal or cutaneous system, or other tissues or systems.

### Example 15 (*: compositions of the invention)

A typical systemic administration by intradermal injection was carried out as follows. A male subject, age 90, injected intradermally on the forearms the following N-acylpeptide derivative of the present invention:
(a). N-Ac-L-Tyr-L-Tyr-L-Tyr-OH, 0.05 ml (0.5 mg) of 1% in water* after 8 days, there was 10-20% increase in skin thickness of 6 mm size skin at the injection site: effective for aging skin.
(b). N-Ac-L-Ala-L-Leu-L-Lys-L-His-L-Arg-NH₂ (SEQ ID NO: 51), 0.05 ml (0.5 mg) of 1% in water.
(c). N-Bz-L-Ala-L-Leu-L-Lys-L-His-L-Arg-NH₂ (SEQ ID NO: 65), 0.05 ml (0.5 mg) of 1% in water.
(d). N-Ac-(EEASPEAVAGVGFESK)-NH₂ (SEQ ID NO: 126), 0.1 ml (0.4 mg) of 0.4% in water.

The intradermal injection of a composition containing an N-acylpeptide derivative of the present invention can be therapeutically effective for systemic treatment of disorders, diseases, symptoms or syndromes associated with tumors, cancers, immune, nervous, vascular, musculoskeletal or cutaneous system, or other tissues or systems.

### Example 17

A male subject, age 41, topically applied a control solution WEP442 on his forehead, but he did not notice any signs of changes for the ensuing several hours. The subject then topically applied N-Ac-L-Tyr-L-Tyr-L-Tyr-OH, 0.2 % in WEP442 on his forehead. After about one hour later, he had an urge for urination, and resulted in discharge of full amount of urine. The subject repeated the same procedure the next day, and obtained the same result.

The N-acylpeptide derivative of the present invention appears to be a diuretic substance, and can be used for treatment of high blood pressure, obesity, overweight or for weight control.

### Example 18

N-Acylpeptide derivative of the present invention can be administered for treatment of knee osteoarthritis by intra-articular injections.

A male subject, age 90, had severe osteoarthritis of both knees for more than four years. Prior therapy had included intra-articular injections of corticosteroids and hyaluronic acid as well as celecoxib (Celebrex) orally (200 mg) twice daily. Such therapy had provided only mild transitory relief of knee pain and edema, and edema of lower legs.

Intra-articular injections of 1% (w/v) N-Ac-L-Tyr-L-Tyr-L-Tyr-OH in water, 0.1 ml (1 mg) as prepared in Example 4 were administered to each knee. The pains in both knees disappeared about 20-30 minutes after the injections, and the relief of pains lasted for 24 hours. Edema and inflammation of the knees and lower legs had diminished for approximately the same 24 hour period. Repeat injections of the same composition were administered once a week for several weeks to provide continued relief of pain, edema and inflammation.

In another trial, intra-articular injections of 0.2% (w/v) N-Ac-L-Tyr-L-Tyr-L-Tyr-OEt with 1% (w/v) methyl cellulose in water, 0.5 ml (1 mg) as prepared in Example 4 were also administered to each knee at different times. The pains in both knees disappeared about 20-30 minutes after the injections, and the relief of pains lasted for 24 hours. Edema and inflammation of the knees and lower legs had diminished for approximately the same 24 hour period.

The above results show that N-acylpeptide derivatives of the present invention can be used of treating inflammation, arthritis, pain, other immune and nerve disorders via systemic administration.

### Example 19

A female subject, age 42, selected a 5 cm x 5 cm skin site on each of her extensor forearms. The subject topically applied three times daily the control vehicle WEP442 on her left extensor forearm, and N-Ac-L-Tyr-L-Tyr-L-Tyr-OEt, 0.8% (w/v) in WEP442 on her right extensor forearm for four weeks. At the end of four weeks, while the skin site in the vehicle control left forearm was still loose, relatively thin and wrinkled, the skin site in the right forearm was palpably raised, smooth, less wrinkled and subtantially increased in skin thickness. While there was no change in the skin site of vehicle control left forearm, the skin site of right forearm treated with N-Ac-L-Tyr-L-Tyr-L-Tyr-OEt had approximately 33% increase in skin thickness as measured by the electronic micrometer caliper.

The above result indicates that N-acylpeptide derivatives of the present invention can be used for topical treatment of disturbed keratinization and aging related changes of skin, nail and hair including fine lines, wrinkles, photoaging, age spots, blotches, mottled skin, stretch marks and for younger-looking skin and skin lightening.

### Example 20

A female subject, age 51, having photoaging skin on her forearms seleted 5 skin test sites with approximately 4 cm x 4 cm in each size, and marked A, B, C, D, and E. The subject topically applied three times daily to test skin sites for four weeks:
A. N-Ac-L-Tyr-L-Tyr-L-Tyr-OEt, 0.5%(w/v) in WEP442
B. N-Ac-L-Tyr-L-Tyr-L-Tyr-OEt, 1%(w/v) in WEP442
C. N-Ac-L-Tyr-L-Tyr-L-Tyr-NH₂, 0.8%(w/v) in WEP442
D. N-Ac-L-Tyr-L-Tyr-L-Tyr-NH₂, 1%(w/v) in WEP442
E. Vehicle control, WEP442

At the end of four weeks, while the skin in the vehicle control site, E was still loose, relatively thin and wrinkled, the skins in test sites A, B, C and D were palpably raised, smooth, less wrinkled and increased in skin thickness. While there was no change in skin thickness of the control site, the treated skins in sites B, C and D had approximately 12%, 15%, and 28% respectively in increased skin thickness as measured by the electronic micrometer caliper.

The above results indicate that N-acylpeptide derivatives of the present invention can be used for topical treatment of disturbed keratinization and aging related changes of skin, nail and hair including fine lines, wrinkles, photoaging, age spots, blotches, mottled skin, stretch marks, and for younger-looking skin and skin lightening.

### Example 21

A female subject, age 52, having sun damaged skin on her forearms seleted 3 skin sites with approximately 5 cm x 5 cm in each size, and marked A,B,C. The subject topically applied three times daily to skin sites for one week:
A. N-Ac-L-Tyr-L-Tyr-L-Tyr-OH, 1%(w/v) in WEP442
B. N-Ac-L-Tyr-L-Tyr-L-Tyr-NH₂, 1%(w/v) in WEP442
C. Vehicle control, WEP442

At the end of one week, while the skin in the vehicle control site C was still loose, relatively thin and wrinkled, the skins in test sites A and B were palpably raised, smooth, less wrinkled and increased in skin thickness. While there was no change in skin thickness of the control site, the treated skins in sites A and B had approximately 29% and 24% respectively in increased skin thickness as measured by the electronic micrometer caliper.

The above results indicate that N-acylpeptide derivatives of the present invention can be used for topical treatment of disturbed keratinization and aging related changes of skin, nail and hair including fine lines, wrinkles, photoaging, age spots, blotches, mottled skin, stretch marks, and for younger-looking skin and skin lightening.

### Example 22

A female subject, age 59, had rosacea on her face with erythema, inflammation and dilated blood vessels, described as telangiectatic rosacea. The subject topically applied twice daily N-Ac-L-Tyr-L-Tyr-L-Tyr-OEt, 1% (w/v) in WEP442 for two weeks. At the end of two weeks, the erythema and inflammation had diminished, and improvement in rduced size of telangiectatic blood vessels was discernible. The rosacea lesions had approximately 25% improvement as judged by clinical evaluation.

The above result indicates that N-acylpeptide derivative of the present invention can be used for topical treatment of rosacea, acne, inflammation and vascular disorders.

### Example 23

A male subject, age 90, had bilateral inter-metacarpal atrophy of both hands with certain restrictions of hand movements. The subject topically applied two to three times daily N-Ac-L-Tyr-L-Tyr-L-Tyr-OEt, 2% (w/v) in EP73 on the back of his left hand, and vehicle control EP73 on the back of his right hand for three weeks. At the end of three weeks, while there was no discernible improvement on his right hand, the inter-metacarpal atrophy of his left hand disappeared almost completely, and the left hand had free movement without restrictions.

The subject now, topically applied twice daily N-Ac-L-Tyr-L-Tyr-L-Tyr-NH₂, 1% (w/v) in WEP442 on the back of his left hand as a mantenace therapy. The left hand continued to improve with no signs of inter-metacarpal atrophy.

The above result indicates that N-acylpeptide derivative of the present invention can be used for topical treatment of rosacea, acne, inflammation and vascular disorders.

### Example 24

Skin thickness study can also be carried out as follows. A male subject, age 90, had moderate photoaging on backs of both hands for many years. Before the study, the skin thickness of his left back hand averaged 1.70 mm, and that of right hand averaged 1.68 mm as measured by the electronic micrometer caliper. The subject topically applied two to three times daily N-Ac-L-Tyr-L-Tyr-L-Tyr-OEt, 2% (w/v) in EP73 on the back of his left hand, and vehicle control EP73 on the back of his right hand for six weeks. At the end of six weeks, while the control skin on the back of his right hand was still loose, relatively thin and wrinkled, the skin on the back of his left hand was palpably raised, smooth, less wrinkled and increased in skin thickness. While the skin thickness on the control back of right hand averaged 1.71 mm, the skin on the back of left hand averaged 2.59 mm; an increase of 52% over the starting skin thickness.

The above result indicates that N-acylpeptide derivative of the present invention can be used for topical treatment of disturbed keratinization and aging related changes of skin, nail and hair including fine lines, wrinkles, photoaging, age spots, blotches, mottled skin, stretch marks, and for younger-looking skin and skin lightening.

### Example 25

A male subject, age 90, had osteoarthritis of both knees for more than four years. For over the past 3 years, repeated courses of intra-articular injections of products containing hyaluronic acid had failed to provide any sustained benefits nor to arrest the progression of symptoms. Intra-articular injections of each knee with N-Ac-L-Tyr-L-Tyr-L-Tyr-OH, 7-13 mg in aqueous solution had provided substantial relief of pain in the joints for 2-3 days.

For maintenance therapy, continued topical applications 2-3 times daily of N-Ac-L-Tyr-L-Tyr-L-Tyr-OEt, 1% (w/v) in EP73 on the skin covering the knee areas had provided continued relief of knee joint pains.

The above results show that N-acyldipeptide derivatives of the present invention can be used for treating inflammation, arthritis, pain, other immune and nerve disorders via systemic or topical administration.

### Supplementary Test Results and Summary

In addition or complementary to the above Examples, additional test results and summary are described in the following sections.

**Volunteer Subjects:** In these studies, the participating subjects are as follows:
Subject 1. Male, age 78, had multiple red and itchy inflammation, dermatitis, or eczema lesions, which were resistant to conventional treatments including corticosteroids.
Subject 2. Female, age 31, had small red and itchy lesions which were resistant to topical corticosteroid treatment.
Subject 3. Female, age 43, had multiple red and itchy inflammation, dermatitis, or eczema lesions over the body for many years, which were resistant to conventional treatments including corticosteroids.
Subject 4. Female, age 50, had red and itchy inflammation, dermatitis, or eczema lesions for many years, which were resistant to topical corticosteroid treatment.
Subject 5. Female, age 41, had early stage of aging related skin changes on both forearms as indicated by age spots and wrinkled skin caused by solar damage.
Subject 6. Female, age 52, had age spots, keratoses and wrinkles on both forearms caused by intrinsic and extrinsic aging.
Subject 7. Female, age 51, had age spots, and wrinkles on both forearms caused by intrinsic and extrinsic aging.
Subject 8. Male, age 90, had osteoarthritis of both knees with inflammation and pain for more than 4 years, and had only mild transitory relief from conventional treatments.
Subject 9. Female, age 41, had sensitive skin with inflammatory lesions on the body.
Subject 12. Male, age 41, had dermatitis and inflammatory lesions on the left palm.
Subject 14. Female, age 73, had multiple age spots including lentigines and keratosis on her face.
Subject 15. Male, age 36, had psorisis
Subject 16. Male, age 80, had sensitive skin and eczema for more than 40 years

Other subjects with various skin and medical conditions and disorders also participated in the present tests and studies.

### Test Methods.

In one embodiment, the test compositions containing N-acylpeptide derivatives of the present invention were tested *in vitro* screen for their biological efficacy in cell cultures as described in Example 5.

In another embodiment, the volunteer subject topically applied the test compositions containing N-acylpeptide derivatives of the present invention on involved skin or lesions once or twice daily for several weeks or until the involved lesions completely cleared and clinically changed to normal skin. As a control study, the subject also topically applied a vehicle control composition on the involved skin or lesions twice daily for the same period.

In yet another embodiment, the volunteer subject topically applied once or twice daily the test compositions containing N-acylpeptide derivatives of the present invention on the skin site above arthritic joints or painful muscles to provide therapeutic effects for the systemic disorders via topical administration.

In yet another embodiment, the volunteer subject injected intra-articularly into a knee joint a test composition containing a N-acylpeptide derivative of the present invention to improve and reduce arthritic inflammation and pain of the joint.

Some test results are summarized as follows.
DK: Disturbed keratinization
AG: Aging related changes of skin, nail and hair
DI: Deranged immune disorders and inflammation
ND: Nerve disorders
CA: Tumors and/or cancers
1+: 25% Efficacy 2+: 50% Efficacy 3+: 75% Efficacy 4+: 95-100% Efficacy

| Tripeptide Derivative | DK | AG | DI | ND |
|---|---|---|---|---|
| N-Ac-L-Tyr-L-Tyr-L-Tyr-OH * | 2+ | 2+ | 3+ | 3+ |
| N-Ac-L-Tyr-L-Tyr-L-Tyr-OEt * | 3+ | 3+ | 3+ | 4+ |
| N-Ac-L-Tyr-L-Tyr-L-Tyr-NH₂ * | 4+ | 4+ | 4+ | 4+ |
| N-Ac-L-Ser-L-Ser-L-Ser-NH₂ | 2+ | 2+ | 2+ | 2+ |
| N-Ac-L-Arg-L-Arg-L-Arg-NH₂ | 2+ | 2+ | 2+ | 2+ |
| N-Ac-L-Val-L-Val-L-Ala-NH₂ | 2+ | 2+ | 2+ | 3+ |
| N-Ac-L-Val-L-Tyr-L-Tyr-NH₂ | 2+ | 3+ | 2+ | 4+ |

| | | | | |
|---|---|---|---|---|
| (*: compositions of the invention) | | | | |

| Pentapeptide Derivative | DK | AG | DI | ND |
|---|---|---|---|---|
| N-Ac-L-Ala-L-Leu-L-Lys-L-His-L-Arg-NH₂ (SEQ ID NO: 5]) | 3+ | 3+ | 4+ | 3+ |
| N-Pr-L-Ala-L-Leu-L-Lys-L-His-L-Arg-NH₂(SEQ ID NO: 58) | 2+ | 2+ | 2+ | 2+ |
| N-Bz-L-Ala-L-Leu-L-Lys-L-His-L-Arg-NH₂ (SEQ ID NO: 65) | 3+ | 4+ | 3+ | 2+ |

| Hexadecapeptide Derivative | CA |
|---|---|
| N-Ac-(EEASPEAVAGVGBESK)-NH₂ (SEQ ID NO: 126) | 9% Inhibition |

The N-acylpeptide derivative of the present invention can be topically administered to provide topical effects or to exert therapeutic effects for systemic diseases. The compositions containing N-acylpeptide derivatives can be used to improve arthritis and pain of joints and muscles, or to reduce, control or lose appetite via topical application. The N-acylpeptide derivative of the present invention can also be given by systemic administration to improve systemic diseases.

As shown in the Examples, the composition containing the N-acylpeptide derivative can be used to improve arthritis of knee joints via intra-articular injection.

The increased skin thickness or plump as shown in the Examples was not due to increased water retention or edema of the skin because the thickness maintained for many months after discontinuation of the treatment. In a publication by Ditre et al. J. Amer Acad Dermatol, 1996, pages 187-195, under "Effects of α-hydroxy acids on photoaged skin: A pilot clinical, histologic, and ultrastructural study", histologic and ultrastructural studies show that skin plump or increased skin thickness caused by topical application of a substance results from a combination of epidermal and dermal changes. In epidermal changes, the epidermis increases in thickness, and the melanin pigmentation shows less clumping of melanin resulting in lighter skin color and improved age spots.
In dermal changes, there are increased amounts of both glycosaminoglycans (GAGs) and collagen fibers, and elastic fibers tend to be longer and thicker.

When a substance is found to plump or increase the skin thickness, those skilled in the art will consider that the substance is reasonably expected or predicted to improve aging related skin changes including fine lines, wrinkles, photoaging, age spots, blotches, mottled skin, and for younger-looking skin and skin lightening.

Therefore, a composition containing N-acylpeptide derivatives of the present invention can be used for topical treatment of aging related skin changes including fine lines, wrinkles, photoaging, age spots, blotches, mottled skin, and for younger-looking skin and skin lightening.

## Claims

1. A composition for topical or systemic administration to a mammal comprising a pharmaceutically or cosmetically acceptable carrier and a therapeutically effective amount of a peptide derivative having the following generic Formula (I):
R₁-AAB-(AAA)ₙ-AAC-R₂ Formula (I)
wherein R₁ is an acyl radical selected from the group consisting of Ac, Pr and Bz; R₂ is selected from the group consisting of OH, OEt, NH₂, NHOH and NHNH₂; AAB is an amino-terminal amino acid residue; (AAA)ₙ is a peptide having n amino acid residues; n is 1; AAC is a carboxyl-terminal amino acid residue; and AAB, AAA, AAC are L-Tyr, L-Tyr, L-Tyr, respectively.

2. The composition of claim 1, wherein the peptide derivative is selected from the group consisting of N-Ac-L-Tyr-L-Tyr-L-Tyr-OH, N-Ac-L-Tyr-L-Tyr-L-Tyr-OEt, and N-Ac-L-Tyr-L-Tyr-L-Tyr-NH₂.

3. The composition of claim 1, wherein the peptide derivative is selected from the group consisting of N-Ac-L-Tyr-L-Tyr-L-Tyr-OH, N-Ac-L-Tyr-L-Tyr-L-Tyr-OEt, and N-Ac-L-Tyr-L-Tyr-L-Tyr-NH₂, for use in treating a disorder selected from disturbed keratinization, aging related changes of skin, hair and nails; deranged immune disorders, inflammation and nerve disorders.

4. Use of the composition of claim 1, wherein the peptide derivative is selected from the group consisting of N-Ac-L-Tyr-L-Tyr-L-Tyr-OH, N-Ac-L-Tyr-L-Tyr-L-Tyr-OEt, and N-Ac-L-Tyr- L-Tyr-L-Tyr-NH₂ for increasing skin thickness or treating dry skin, mottled skin, fine lines, wrinkles, age spots or lentigine.

5. The composition for use of claim 3, wherein the disorder is selected from the group consisting of erythema, rosacea, psoriasis, dermatitis, eczema, itch, photoaging, acne, arthritis and pain.

6. A composition comprising a peptide derivative for use in therapy having the following generic Formula (I):
R₁-AAB-(AAA)ₙ-AAC-R₂ Formula (I)
wherein R₁ is an acyl radical selected from the group consisting of Ac, Pr and Bz; R₂ is selected from the group consisting of OH, OEt, NH₂, NHOH and NHNH₂; AAB is an amino-terminal amino acid residue; (AAA)ₙ is a peptide having n amino acid residues; n is 1; AAC is a carboxyl-terminal amino acid residue; wherein AAB, AAA, AAC are L-Tyr, L-Tyr, L-Tyr, respectively.

## Patentansprüche

1. Eine Zusammensetzung zur topischen oder systemischen Verabreichung an ein Säugetier, umfassend einen pharmazeutischen oder kosmetisch verträglichen Träger und eine therapeutisch effektive Menge eines Peptidderivats mit der folgenden generischen Formel (I):
R₁-AAB-(AAA)ₙ-AAC-R₂ Formel (I),
wobei R₁ ein Acylradikal ist, ausgewählt aus der Gruppe, bestehend aus Ac, Pr und Bz; R₂ ausgewählt ist aus der Gruppe, bestehend aus OH, OEt, NH₂, NHOH und NHNH₂; AAB ein Amino-terminaler Aminosäurerest ist; (AAA)ₙ ein Peptid ist mit n-Aminosäureresten; n gleich 1 ist; AAC ein Carboxyl-terminaler Aminosäurerest ist; und AAB, AAA, AAC jeweils L-Tyr, L-Tyr, L-Tyr sind.

2. Die Zusammensetzung nach Anspruch 1, wobei das Peptidderivat ausgewählt ist aus der Gruppe, bestehend aus N-AcL-Tyr-L-Tyr-L-Tyr-OH, N-Ac-L-Tyr-L-Tyr-L-Tyr-OEt und N-Ac-L-Tyr-L-Tyr-L-Tyr-NH₂.

3. Die Zusammensetzung nach Anspruch 1, wobei das Peptidderivat ausgewählt ist aus der Gruppe, bestehend aus N-AcL-Tyr-L-Tyr-L-Tyr-OH, N-Ac-L-Tyr-L-Tyr-L-Tyr-OEt und N-Ac-L-Tyr-L-Tyr-L-Tyr-NH₂ zur Verwendung in der Behandlung einer Erkrankung, ausgewählt aus gestörter Keratinisierung, altersbedingte Veränderung in der Haut, Haare, Nägel; Zustände von gestörtem Immunsystem, Enzündung und Nervenerkränkungen.

4. Die Verwendung der Zusammensetzung von Anspruch 1, wobei das Peptidderivat ausgewählt ist aus der Gruppe, bestehend aus N-AcL-Tyr-L-Tyr-L-Tyr-OH, N-Ac-L-Tyr-L-Tyr-L-Tyr-OEt und N-Ac-L-Tyr-L-Tyr-L-Tyr-NH₂ zur Verbesserung der Hautdicke oder Behandlung von trockener Haut, fleckiger Haut, feinen Linien, Falten, Altersflecken oder Lentigo.

5. Die Zusammensetzung zur Verwendung von Anspruch 3, wobei die Erkrankung ausgewählt ist aus der Gruppe, bestehend aus Erythem, Rosacea, Psoriasis, Dermatitis, Ekzem, Juckreiz, Photoalterung, Akne, Arthritis und Schmerz.

6. Die Zusammensetzung, umfassend ein Peptidderivat zur Verwendung in der Therapie mit der folgenden generischen Formel (I):
R₁-AAB-(AAA)ₙ-AAC-R₂ Formel (I),
wobei R₁ ein Acylradikal ist, ausgewählt aus der Gruppe, bestehend aus Ac, Pr und Bz; R₂ ausgewählt ist aus der Gruppe, bestehend aus OH, OEt, NH₂, NHOH und NHNH₂; AAB ein Amino-terminaler Aminosäurerest ist; (AAA)ₙ ein Peptid ist mit n-Aminosäureresten; n gleich 1 ist; AAC ein Carboxyl-terminaler Aminosäurerest ist; wobei AAB, AAA, AAC jeweils L-Tyr, L-Tyr, L-Tyr sind.

## Revendications

1. Composition pour administration topique ou systémique à un mammifère comprenant un véhicule acceptable sur le plan pharmaceutique ou cosmétique et une quantité efficace sur le plan thérapeutique d'un dérivé peptidique ayant la formule générique (I) suivante :
R₁-AAB-(AAA)ₙ-AAC-R₂ Formule (I)
dans laquelle R₁ est un radical acyle choisi dans le groupe constitué de Ac, Pr et Bz ; R₂ est choisi dans le groupe constitué de OH, OEt, NH₂, NHOH et NHNH₂ ; AAB est un résidu d'acide aminé à terminaison amino ; (AAA)ₙ est un peptide ayant n résidus d'acide aminé ; n vaut 1 ; AAC est un résidu d'acide aminé à terminaison carboxyle ; et AAB, AAA, AAC sont respectivement L-Tyr, L-Tyr et L-Tyr.

2. Composition selon la revendication 1, le dérivé peptidique étant choisi dans le groupe constitué de N-Ac-L-Tyr-L-Tyr-L-Tyr-OH, N-Ac-L-Tyr-L-Tyr-L-Tyr-OEt et N-Ac-L-Tyr-L-Tyr-L-Tyr-NH₂.

3. Composition selon la revendication 1, le dérivé peptidique étant choisi dans le groupe constitué de N-Ac-L-Tyr-L-Tyr-L-Tyr-OH, N-Ac-L-Tyr-L-Tyr-L-Tyr-OEt et N-Ac-L-Tyr-L-Tyr-L-Tyr-NH₂, destinée à être utilisée pour traiter un trouble choisi parmi une kératinisation anormale, des modifications de la peau, des cheveux et des ongles liées à l'âge ; des troubles liés à une immunité perturbée, une inflammation et des troubles nerveux.

4. Utilisation de la composition selon la revendication 1, le dérivé peptidique étant choisi dans le groupe constitué de N-Ac-L-Tyr-L-Tyr-L-Tyr-OH, N-Ac-L-Tyr-L-Tyr-L-Tyr-OEt et N-Ac-L-Tyr-L-Tyr-L-Tyr-NH₂, pour augmenter l'épaisseur de la peau ou traiter la peau sèche, la peau marbrée, les ridules, les rides, les tâches de vieillesse ou le lentigo.

5. Composition destinée à être utilisée selon la revendication 3, le trouble étant choisi dans le groupe constitué d'érythème, rosacée, psoriasis, dermatite, eczéma, démangeaisons, photovieillissement, acné, arthrite et douleurs.

6. Composition comprenant un dérivé peptidique pour utilisation à des fins thérapeutiques ayant la formule générique (I) suivante :
R₁-AAB-(AAA)ₙ-AAC-R₂ Formule (I)
dans laquelle R₁ est un radical acyle choisi dans le groupe constitué de Ac, Pr et Bz ; R₂ est choisi dans le groupe constitué de OH, OEt, NH₂, NHOH et NHNH₂ ; AAB est un résidu d'acide aminé à terminaison amino ; (AAA)ₙ est un peptide ayant n résidus d'acide aminé ; n vaut 1 ; AAC est un résidu d'acide aminé à terminaison carboxyle ; et AAB, AAA, AAC sont respectivement L-Tyr, L-Tyr et L-Tyr.


### SEQUENCE LISTING

<110> Yu, Ruey and Eugene Van Scott
<120> N-acylpeptide Derivatives and Their Uses
<130> 208976-315WO
<160> 174
<170> PatentIn version 3.5
<210> 1
   <211> 4
   <212> PRT
   <213> Ovibos moschatus
<400> 1
   Glu Met Cys Gly
   1
<210> 2
   <211> 9
   <212> PRT
   <213> Homo sapiens
<220>
   <221> MOD_RES
   <222> (1)..(1)
   <223> modified by Pyro
<220>
   <221> MOD_RES
   <222> (9)..(9)
   <223> modified by NH2
<400> 2
   Glu His Trp Ser Tyr Gly Leu Arg Pro
   1               5
<210> 3
   <211> 15
   <212> PRT
   <213> Homo sapiens
<400> 3
   Glu Ala Ser Pro Glu Ala Val Ala Gly Val Gly Phe Glu Ser Lys
   1               5                   10                  15
<210> 4
   <211> 16
   <212> PRT
   <213> Homo sapiens
<400> 4
   Glu Glu Ala Ser Pro Glu Ala Val Ala Gly Val Gly Phe Glu Ser Lys
   1               5                   10                  15
<210> 5
   <211> 6
   <212> PRT
   <213> Homo sapiens
<400> 5
   Cys Ser Val Thr Cys Gly
   1               5
<210> 6
   <211> 20
   <212> PRT
   <213> Homo sapiens
<400> 6
   Phe Cys Thr Gly Ile Arg Val Ala His Leu Ala Leu Lys His Arg Gln
   1               5                   10                  15
   Gly Lys Asn His
               20
<210> 7
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by OH
<400> 7
   Tyr Tyr Tyr Tyr
   1
<210> 8
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by OEt
<400> 8
   Tyr Tyr Tyr Tyr
   1
<210> 9
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by NH2
<400> 9
   Tyr Tyr Tyr Tyr
   1
<210> 10
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by NHAc
<400> 10
   Tyr Tyr Tyr Tyr
   1
<210> 11
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by NHNH2
<400> 11
   Tyr Tyr Tyr Tyr
   1
<210> 12
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by NHNHAc
<400> 12
   Tyr Tyr Tyr Tyr
   1
<210> 13
   <211> 4
   <212> PRT
   <213> Artificial Sequence
<220>
   <223> N-acyltetrapeptide derivative
<220>
   <221> MOD_RES
   <222> (1)..(1)
   <223> modified by Ac
<220>
   <221> MOD_RES
   <222> (4)..(4)
   <223> modified by NHOH
<400> 13
   Tyr Tyr Tyr Tyr
   1
<210> 14
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by OH
<400> 14
   Tyr Tyr Tyr Tyr
   1
<210> 15
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by OEt
<400> 15
   Tyr Tyr Tyr Tyr
   1
<210> 16
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by NH2
<400> 16
   Tyr Tyr Tyr Tyr
   1
<210> 17
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by NHPr
<400> 17
   Tyr Tyr Tyr Tyr
   1
<210> 18
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by NHNH2
<400> 18
   Tyr Tyr Tyr Tyr
   1
<210> 19
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by NHNHPr
<400> 19
   Tyr Tyr Tyr Tyr
   1
<210> 20
   <211> 4
   <212> PRT
   <213> Artificial Sequence
<220>
   <223> N-acyltetrapeptide derivative
<220>
   <221> MOD_RES
   <222> (1)..(1)
   <223> modified by Pr
<220>
   <221> MOD_RES
   <222> (4)..(4)
   <223> modified by NHOH
<400> 20
   Tyr Tyr Tyr Tyr
   1
<210> 21
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by OH
<400> 21
   Tyr Tyr Tyr Tyr
   1
<210> 22
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by OEt
<400> 22
   Tyr Tyr Tyr Tyr
   1
<210> 23
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by NH2
<400> 23
   Tyr Tyr Tyr Tyr
   1
<210> 24
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by NHBz
<400> 24
   Tyr Tyr Tyr Tyr
   1
<210> 25
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by NHNH2
<400> 25
   Tyr Tyr Tyr Tyr
   1
<210> 26
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by NHNHBz
<400> 26
   Tyr Tyr Tyr Tyr
   1
<210> 27
   <211> 4
   <212> PRT
   <213> Artificial Sequence
<220>
   <223> N-acyltetrapeptide derivative
<220>
   <221> MOD_RES
   <222> (1)..(1)
   <223> modified by Bz
<220>
   <221> MOD_RES
   <222> (4)..(4)
   <223> modified by NHOH
<400> 27
   Tyr Tyr Tyr Tyr
   1
<210> 28
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by OH
<400> 28
   Tyr Tyr Tyr Tyr Tyr
   1               5
<210> 29
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by OEt
<400> 29
   Tyr Tyr Tyr Tyr Tyr
   1 5
<210> 30
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NH2
<400> 30
   Tyr Tyr Tyr Tyr Tyr
   1 5
<210> 31
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHAc
<400> 31
   Tyr Tyr Tyr Tyr Tyr
   1 5
<210> 32
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHNH2
<400> 32
   Tyr Tyr Tyr Tyr Tyr
   1               5
<210> 33
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHNHAc
<400> 33
   Tyr Tyr Tyr Tyr Tyr
   1               5
<210> 34
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHOH
<400> 34
   Tyr Tyr Tyr Tyr Tyr
   1 5
<210> 35
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by OH
<400> 35
   Tyr Tyr Tyr Tyr Tyr
   1 5
<210> 36
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by OEt
<400> 36
   Tyr Tyr Tyr Tyr Tyr
   1 5
<210> 37
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NH2
<400> 37
   Tyr Tyr Tyr Tyr Tyr
   1               5
<210> 38
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHPr
<400> 38
   Tyr Tyr Tyr Tyr Tyr
   1               5
<210> 39
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHNH2
<400> 39
   Tyr Tyr Tyr Tyr Tyr
   1 5
<210> 40
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHNHPr
<400> 40
   Tyr Tyr Tyr Tyr Tyr
   1 5
<210> 41
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHOH
<400> 41
   Tyr Tyr Tyr Tyr Tyr
   1 5
<210> 42
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by OH
<400> 42
   Gly Ile Arg Val Ala
   1               5
<210> 43
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by OEt
<400> 43
   Gly Ile Arg Val Ala
   1               5
<210> 44
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NH2
<400> 44
   Gly Ile Arg Val Ala
   1 5
<210> 45
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHAc
<400> 45
   Gly Ile Arg Val Ala
   1 5
<210> 46
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHNH2
<400> 46
   Gly Ile Arg Val Ala
   1 5
<210> 47
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHNHAc
<400> 47
   Gly Ile Arg Val Ala
   1               5
<210> 48
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHOH
<400> 48
   Gly Ile Arg Val Ala
   1               5
<210> 49
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by OH
<400> 49
   Ala Leu Lys His Arg
   1 5
<210> 50
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by OEt
<400> 50
   Ala Leu Lys His Arg
   1 5
<210> 51
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NH2
<400> 51
   Ala Leu Lys His Arg
   1 5
<210> 52
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHAc
<400> 52
   Ala Leu Lys His Arg
   1               5
<210> 53
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHNH2
<400> 53
   Ala Leu Lys His Arg
   1               5
<210> 54
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHNHAc
<400> 54
   Ala Leu Lys His Arg
   1 5
<210> 55
   <211> 5
   <212> PRT
   <213> Artificial Sequence
<220>
   <223> N-acylpentapeptide derivative
<220>
   <221> MOD_RES
   <222> (1)..(1)
   <223> modified by Ac
<220>
   <221> MOD_RES
   <222> (5)..(5)
   <223> modified by NHOH
<400> 55
   Ala Leu Lys His Arg
   1 5
<210> 56
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by OH
<400> 56
   Ala Leu Lys His Arg
   1 5
<210> 57
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by OEt
<400> 57
   Ala Leu Lys His Arg
   1               5
<210> 58
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NH2
<400> 58
   Ala Leu Lys His Arg
   1               5
<210> 59
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHPr
<400> 59
   Ala Leu Lys His Arg
   1               5
<210> 60
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHNH2
<400> 60
   Ala Leu Lys His Arg
   1               5
<210> 61
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHNHPr
<400> 61
   Ala Leu Lys His Arg
   1               5
<210> 62
   <211> 5
   <212> PRT
   <213> Artificial Sequence
<220>
   <223> N-acylpentapeptide derivative
<220>
   <221> MOD_RES
   <222> (1)..(1)
   <223> modified by Pr
<220>
   <221> MOD_RES
   <222> (5)..(5)
   <223> modified by NHOH
<400> 62
   Ala Leu Lys His Arg
   1               5
<210> 63
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by OH
<400> 63
   Ala Leu Lys His Arg
   1               5
<210> 64
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by OEt
<400> 64
   Ala Leu Lys His Arg
   1 5
<210> 65
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by NH2
<400> 65
   Ala Leu Lys His Arg
   1 5
<210> 66
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by NHBz
<400> 66
   Ala Leu Lys His Arg
   1 5
<210> 67
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by NHNH2
<400> 67
   Ala Leu Lys His Arg
   1               5
<210> 68
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by NHNHPr
<400> 68
   Ala Leu Lys His Arg
   1               5
<210> 69
   <211> 5
   <212> PRT
   <213> Artificial Sequence
<220>
   <223> N-acylpentapeptide derivative
<220>
   <221> MOD_RES
   <222> (1)..(1)
   <223> modified by Bz
<220>
   <221> MOD_RES
   <222> (5)..(5)
   <223> modified by NHOH
<400> 69
   Ala Leu Lys His Arg
   1 5
<210> 70
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by OH
<400> 70
   Tyr Tyr Tyr Tyr Tyr Tyr
   1 5
<210> 71
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by OEt
<400> 71
   Tyr Tyr Tyr Tyr Tyr Tyr
   1 5
<210> 72
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NH2
<400> 72
   Tyr Tyr Tyr Tyr Tyr Tyr
   1               5
<210> 73
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHAc
<400> 73
   Tyr Tyr Tyr Tyr Tyr Tyr
   1               5
<210> 74
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHNH2
<400> 74
   Tyr Tyr Tyr Tyr Tyr Tyr
   1 5
<210> 75
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHNHAc
<400> 75
   Tyr Tyr Tyr Tyr Tyr Tyr
   1 5
<210> 76
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHOH
<400> 76
   Tyr Tyr Tyr Tyr Tyr Tyr
   1 5
<210> 77
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by OH
<400> 77
   Tyr Tyr Tyr Tyr Tyr Tyr
   1               5
<210> 78
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by OEt
<400> 78
   Tyr Tyr Tyr Tyr Tyr Tyr
     1               5
<210> 79
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NH2
<400> 79
   Tyr Tyr Tyr Tyr Tyr Tyr
   1               5
<210> 80
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHPr
<400> 80
   Tyr Tyr Tyr Tyr Tyr Tyr
   1               5
<210> 81
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHNH2
<400> 81
   Tyr Tyr Tyr Tyr Tyr Tyr
   1               5
<210> 82
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHNHPr
<400> 82
   Tyr Tyr Tyr Tyr Tyr Tyr
   1               5
<210> 83
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHOH
<400> 83
   Tyr Tyr Tyr Tyr Tyr Tyr
   1               5
<210> 84
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by OH
<400> 84
   Tyr Tyr Tyr Tyr Tyr Tyr
   1 5
<210> 85
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by OEt
<400> 85
   Tyr Tyr Tyr Tyr Tyr Tyr
   1 5
<210> 86
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by NH2
<400> 86
   Tyr Tyr Tyr Tyr Tyr Tyr
   1 5
<210> 87
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by NHBz
<400> 87
   Tyr Tyr Tyr Tyr Tyr Tyr
   1               5
<210> 88
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by NHNH2
<400> 88
   Tyr Tyr Tyr Tyr Tyr Tyr
   1               5
<210> 89
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by NHNHBz
<400> 89
   Tyr Tyr Tyr Tyr Tyr Tyr
   1 5
<210> 90
   <211> 6
   <212> PRT
   <213> Artificial Sequence
<220>
   <223> N-acylhexapeptide derivative
<220>
   <221> MOD_RES
   <222> (1)..(1)
   <223> modified by Bz
<220>
   <221> MOD_RES
   <222> (6)..(6)
   <223> modified by NHOH
<400> 90
   Tyr Tyr Tyr Tyr Tyr Tyr
   1 5
<210> 91
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by OH
<400> 91
   Cys Ser Val Thr Cys Gly
   1 5
<210> 92
   <211> 6
   <212> PRT
   <213> Artificial Sequence
<220>
   <223> N-acylhexapeptide derivative
<220>
   <221> MOD_RES
   <222> (1)..(1)
   <223> modifid by Ac
<220>
   <221> MOD_RES
   <222> (6)..(6)
   <223> modifid by OEt
<400> 92
   Cys Ser Val Thr Cys Gly
   1               5
<210> 93
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NH2
<400> 93
   Cys Ser Val Thr Cys Gly
   1               5
<210> 94
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHAc
<400> 94
   Cys Ser Val Thr Cys Gly
   1 5
<210> 95
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHNH2
<400> 95
   Cys Ser Val Thr Cys Gly
   1 5
<210> 96
   <211> 6
   <212> PRT
   <213> Artificial Sequence
<220>
   <223> N-acylhexapeptide derivative
<220>
   <221> MOD_RES
   <222> (1)..(1)
   <223> modified by Ac
<220>
   <221> MOD_RES
   <222> (6)..(6)
   <223> modified by NHNHAc
<400> 96
   Cys Ser Val Thr Cys Gly
   1 5
<210> 97
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by OH
<400> 97
   Cys Ser Val Thr Cys Gly
   1               5
<210> 98
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by OEt
<400> 98
   Cys Ser Val Thr Cys Gly
   1               5
<210> 99
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NH2
<400> 99
   Cys Ser Val Thr Cys Gly
   1 5
<210> 100
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHPr
<400> 100
   Cys Ser Val Thr Cys Gly
   1 5
<210> 101
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHNH2
<400> 101
   Cys Ser Val Thr Cys Gly
   1 5
<210> 102
   <211> 6
   <212> PRT
   <213> Artificial Sequence
<220>
   <223> N-acylhexapeptide derivative
<220>
   <221> MOD_RES
   <222> (1)..(1)
   <223> modified by Pr
<220>
   <221> MOD_RES
   <222> (6)..(6)
   <223> modified by NHNHPr
<400> 102
   Cys Ser Val Thr Cys Gly
   1               5
<210> 103
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by OH
<400> 103
   Glu Ala Ser Pro Glu Ala Val Ala Gly Val Gly Phe Glu Ser Lys
   1               5                   10                  15
<210> 104
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by OEt
<400> 104
   Glu Ala Ser Pro Glu Ala Val Ala Gly Val Gly Phe Glu Ser Lys
   1 5 10 15
<210> 105
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by NH2
<400> 105
   Glu Ala Ser Pro Glu Ala Val Ala Gly Val Gly Phe Glu Ser Lys
   1 5 10 15
<210> 106
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by NHAc
<400> 106
   Glu Ala Ser Pro Glu Ala Val Ala Gly Val Gly Phe Glu Ser Lys
   1 5 10 15
<210> 107
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by NHNH2
<400> 107
   Glu Ala Ser Pro Glu Ala Val Ala Gly Val Gly Phe Glu Ser Lys
   1               5                   10                  15
<210> 108
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by NHNHAc
<400> 108
   Glu Ala Ser Pro Glu Ala Val Ala Gly Val Gly Phe Glu Ser Lys
   1               5                   10                  15
<210> 109
   <211> 15
   <212> PRT
   <213> Artificial Sequence
<220>
   <223> N-acylpentadecapeptide derivative
<220>
   <221> MOD_RES
   <222> (1)..(1)
   <223> modified by Ac
<220>
   <221> MOD_RES
   <222> (15)..(15)
   <223> modified by NHOH
<400> 109
   Glu Ala Ser Pro Glu Ala Val Ala Gly Val Gly Phe Glu Ser Lys
   1 5 10 15
<210> 110
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by OH
<400> 110
   Glu Ala Ser Pro Glu Ala Val Ala Gly Val Gly Phe Glu Ser Lys
   1 5 10 15
<210> 111
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by OEt
<400> 111
   Glu Ala Ser Pro Glu Ala Val Ala Gly Val Gly Phe Glu Ser Lys
   1 5 10 15
<210> 112
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by NH2
<400> 112
   Glu Ala Ser Pro Glu Ala Val Ala Gly Val Gly Phe Glu Ser Lys
   1               5                   10                  15
<210> 113
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by NHPr
<400> 113
   Glu Ala Ser Pro Glu Ala Val Ala Gly Val Gly Phe Glu Ser Lys
   1               5                   10                  15
<210> 114
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by NHNH2
<400> 114
   Glu Ala Ser Pro Glu Ala Val Ala Gly Val Gly Phe Glu Ser Lys
   1 5 10 15
<210> 115
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by NHNHPr
<400> 115
   Glu Ala Ser Pro Glu Ala Val Ala Gly Val Gly Phe Glu Ser Lys
   1 5 10 15
<210> 116
   <211> 15
   <212> PRT
   <213> Artificial Sequence
<220>
   <223> N-acylpentadecapeptide derivative
<220>
   <221> MOD_RES
   <222> (1)..(1)
   <223> modified by Pr
<220>
   <221> MOD_RES
   <222> (15)..(15)
   <223> modified by NHOH
<400> 116
   Glu Ala Ser Pro Glu Ala Val Ala Gly Val Gly Phe Glu Ser Lys
   1 5 10 15
<210> 117
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by OH
<400> 117
   Glu Ala Ser Pro Glu Ala Val Ala Gly Val Gly Phe Glu Ser Lys
   1               5                   10                  15
<210> 118
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by OEt
<400> 118
   Glu Ala Ser Pro Glu Ala Val Ala Gly Val Gly Phe Glu Ser Lys
   1               5                   10                  15
<210> 119
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by NH2
<400> 119
   Glu Ala Ser Pro Glu Ala Val Ala Gly Val Gly Phe Glu Ser Lys
   1 5 10 15
<210> 120
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by NHBz
<400> 120
   Glu Ala Ser Pro Glu Ala Val Ala Gly Val Gly Phe Glu Ser Lys
   1 5 10 15
<210> 121
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by NHNH2
<400> 121
   Glu Ala Ser Pro Glu Ala Val Ala Gly Val Gly Phe Glu Ser Lys
   1 5 10 15
<210> 122
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by NHNHBz
<400> 122
   Glu Ala Ser Pro Glu Ala Val Ala Gly Val Gly Phe Glu Ser Lys
   1               5                   10                  15
<210> 123
   <211> 15
   <212> PRT
   <213> Artificial Sequence
<220>
   <223> N-acylpentadecapeptide derivative
<220>
   <221> MOD_RES
   <222> (1)..(1)
   <223> modified by Bz
<220>
   <221> MOD_RES
   <222> (15)..(15)
   <223> modified by NHOH
<400> 123
   Glu Ala Ser Pro Glu Ala Val Ala Gly Val Gly Phe Glu Ser Lys
   1               5                   10                  15
<210> 124
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by OH
<400> 124
   Glu Glu Ala Ser Pro Glu Ala Val Ala Gly Val Gly Phe Glu Ser Lys
   1               5                   10                  15
<210> 125
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by OEt
<400> 125
   Glu Glu Ala Ser Pro Glu Ala Val Ala Gly Val Gly Phe Glu Ser Lys
   1               5                   10                  15
<210> 126
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by NH2
<400> 126
   Glu Glu Ala Ser Pro Glu Ala Val Ala Gly Val Gly Phe Glu Ser Lys
   1               5                   10                  15
<210> 127
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by NHAc
<400> 127
   Glu Glu Ala Ser Pro Glu Ala Val Ala Gly Val Gly Phe Glu Ser Lys
   1               5                   10                  15
<210> 128
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by NHNH2
<400> 128
   Glu Glu Ala Ser Pro Glu Ala Val Ala Gly Val Gly Phe Glu Ser Lys
   1               5                   10                  15
<210> 129
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by NHNHAc
<400> 129
   Glu Glu Ala Ser Pro Glu Ala Val Ala Gly Val Gly Phe Glu Ser Lys
   1 5 10 15
<210> 130
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (16)..(16)
   <223> modified by NHOH
<400> 130
   Glu Glu Ala Ser Pro Glu Ala Val Ala Gly Val Gly Phe Glu Ser Lys
   1 5 10 15
<210> 131
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by OH
<400> 131
   Glu Glu Ala Ser Pro Glu Ala Val Ala Gly Val Gly Phe Glu Ser Lys
   1 5 10 15
<210> 132
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by OEt
<400> 132
   Glu Glu Ala Ser Pro Glu Ala Val Ala Gly Val Gly Phe Glu Ser Lys
   1               5                   10                  15
<210> 133
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by NH2
<400> 133
   Glu Glu Ala Ser Pro Glu Ala Val Ala Gly Val Gly Phe Glu Ser Lys
   1               5                   10                  15
<210> 134
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by NHPr
<400> 134
   Glu Glu Ala Ser Pro Glu Ala Val Ala Gly Val Gly Phe Glu Ser Lys
   1               5                   10                  15
<210> 135
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by NHNH2
<400> 135
   Glu Glu Ala Ser Pro Glu Ala Val Ala Gly Val Gly Phe Glu Ser Lys
   1               5                   10                  15
<210> 136
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by NHNHPr
<400> 136
   Glu Glu Ala Ser Pro Glu Ala Val Ala Gly Val Gly Phe Glu Ser Lys
   1               5                   10                  15
<210> 137
   <211> 16
   <212> PRT
   <213> Artificial Sequence
<220>
   <223> N-acylhexadecapeptide derivative
<220>
   <221> MOD_RES
   <222> (1)..(1)
   <223> modified by Pr
<220>
   <221> MOD_RES
   <222> (16)..(16)
   <223> modified by NHOH
<400> 137
   Glu Glu Ala Ser Pro Glu Ala Val Ala Gly Val Gly Phe Glu Ser Lys
   1               5                   10                  15
<210> 138
   <211> 16
   <212> PRT
   <213> Artificial Sequence
<220>
   <223> N-acylhexadecapeptide derivative
<220>
   <221> MOD_RES
   <222> (1)..(1)
   <223> modified by Bz
<220>
   <221> MOD_RES
   <222> (16)..(16)
   <223> modified by OH
<400> 138
   Glu Glu Ala Ser Pro Glu Ala Val Ala Gly Val Gly Phe Glu Ser Lys
   1               5                   10                  15
<210> 139
   <211> 16
   <212> PRT
   <213> Artificial Sequence
<220>
   <223> N-acylhexadecapeptide derivative
<220>
   <221> MOD_RES
   <222> (1)..(1)
   <223> modified by Bz
<220>
   <221> MOD_RES
   <222> (16)..(16)
   <223> modified by OEt
<400> 139
   Glu Glu Ala Ser Pro Glu Ala Val Ala Gly Val Gly Phe Glu Ser Lys
   1 5 10 15
<210> 140
   <211> 16
   <212> PRT
   <213> Artificial Sequence
<220>
   <223> N-acylhexadecapeptide derivative
<220>
   <221> MOD_RES
   <222> (1)..(1)
   <223> modified by Bz
<220>
   <221> MOD_RES
   <222> (16)..(16)
   <223> modified by NH2
<400> 140
   Glu Glu Ala Ser Pro Glu Ala Val Ala Gly Val Gly Phe Glu Ser Lys
   1 5 10 15
<210> 141
   <211> 16
   <212> PRT
   <213> Artificial Sequence
<220>
   <223> N-acylhexadecapeptide derivative
<220>
   <221> MOD_RES
   <222> (1)..(1)
   <223> modified by Bz
<220>
   <221> MOD_RES
   <222> (16)..(16)
   <223> modified by NHBz
<400> 141
   Glu Glu Ala Ser Pro Glu Ala Val Ala Gly Val Gly Phe Glu Ser Lys
   1 5 10 15
<210> 142
   <211> 16
   <212> PRT
   <213> Artificial Sequence
<220>
   <223> artificial sequence
<220>
   <221> MOD_RES
   <222> (1)..(1)
   <223> modified by Bz
<220>
   <221> MOD_RES
   <222> (16)..(16)
   <223> modified by NHNH2
<400> 142
   Glu Glu Ala Ser Pro Glu Ala Val Ala Gly Val Gly Phe Glu Ser Lys
   1               5                   10                  15
<210> 143
   <211> 16
   <212> PRT
   <213> Artificial Sequence
<220>
   <223> N-acylhexadecapeptide derivative
<220>
   <221> MOD_RES
   <222> (1)..(1)
   <223> modified by Bz
<220>
   <221> MOD_RES
   <222> (16)..(16)
   <223> modified by NHNHBz
<400> 143
   Glu Glu Ala Ser Pro Glu Ala Val Ala Gly Val Gly Phe Glu Ser Lys
   1               5                   10                  15
<210> 144
   <211> 16
   <212> PRT
   <213> Artificial Sequence
<220>
   <223> N-acylhexadecapeptide derivative
<220>
   <221> MOD_RES
   <222> (1)..(1)
   <223> modified by Bz
<220>
   <221> MOD_RES
   <222> (16)..(16)
   <223> modified by NHOH
<400> 144
   Glu Glu Ala Ser Pro Glu Ala Val Ala Gly Val Gly Phe Glu Ser Lys
   1               5                   10                  15
<210> 145
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (13)..(13)
   <223> represents Pgly
<220>
   <221> MOD_RES
   <222> (16)..(16)
   <223> modified by NH2
<400> 145
   Glu Glu Ala Ser Pro Glu Ala Val Ala Gly Val Gly Xaa Glu Ser Lys
   1               5                   10                  15
<210> 146
   <211> 16
   <212> PRT
   <213> Artificial Sequence
<220>
   <223> N-acylhexadecapeptide derivative
<220>
   <221> MOD_RES
   <222> (1)..(1)
   <223> modified by Ac
<220>
   <221> MOD_RES
   <222> (13)..(13)
   <223> represents Pgly
<220>
   <221> MOD_RES
   <222> (16)..(16)
   <223> modified by NHNH2
<400> 146
   Glu Glu Ala Ser Pro Glu Ala Val Ala Gly Val Gly Xaa Glu Ser Lys
   1 5 10 15
<210> 147
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by OH
<400> 147
   Cys Lys Lys Glu Glu Ala Ser Pro Glu Ala Val Ala Gly Val Gly Phe
   1 5 10 15
   Glu Ser Lys
<210> 148
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by OEt
<400> 148
   Cys Lys Lys Glu Glu Ala Ser Pro Glu Ala Val Ala Gly Val Gly Phe
   1 5 10 15
   Glu Ser Lys
<210> 149
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by NH2
<400> 149
   Cys Lys Lys Glu Glu Ala Ser Pro Glu Ala Val Ala Gly Val Gly Phe
   1               5                   10                  15
   Glu Ser Lys
<210> 150
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by NHAc
<400> 150
   Cys Lys Lys Glu Glu Ala Ser Pro Glu Ala Val Ala Gly Val Gly Phe
   1               5                   10                  15
   Glu Ser Lys
<210> 151
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by NHNH2
<400> 151
   Cys Lys Lys Glu Glu Ala Ser Pro Glu Ala Val Ala Gly Val Gly Phe
   1               5                   10                  15
   Glu Ser Lys
<210> 152
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by NHNHAc
<400> 152
   Cys Lys Lys Glu Glu Ala Ser Pro Glu Ala Val Ala Gly Val Gly Phe
   1               5                   10                  15
   Glu Ser Lys
<210> 153
   <211> 19
   <212> PRT
   <213> Artificial Sequence
<220>
   <223> N-acylnonadecapeptide derivative
<220>
   <221> MOD_RES
   <222> (1)..(1)
   <223> modified by Ac
<220>
   <221> MOD_RES
   <222> (19)..(19)
   <223> modified by NHOH
<400> 153
   Cys Lys Lys Glu Glu Ala Ser Pro Glu Ala Val Ala Gly Val Gly Phe
   1               5                   10                  15
   Glu Ser Lys
<210> 154
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by OH
<400> 154
   Phe Cys Thr Gly Ile Arg Val Ala His Leu Ala Leu Lys His Arg Gln
   1               5                   10                  15
   Gly Lys Asn His
               20
<210> 155
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by OEt
<400> 155
   Phe Cys Thr Gly Ile Arg Val Ala His Leu Ala Leu Lys His Arg Gln
   1 5 10 15
   Gly Lys Asn His
   20
<210> 156
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by NH2
<400> 156
   Phe Cys Thr Gly Ile Arg Val Ala His Leu Ala Leu Lys His Arg Gln
   1 5 10 15
   Gly Lys Asn His
   20
<210> 157
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by NHAc
<400> 157
   Phe Cys Thr Gly Ile Arg Val Ala His Leu Ala Leu Lys His Arg Gln
   1               5                   10                  15
   Gly Lys Asn His
               20
<210> 158
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by NHNH2
<400> 158
   Phe Cys Thr Gly Ile Arg Val Ala His Leu Ala Leu Lys His Arg Gln
   1               5                   10                  15
   Gly Lys Asn His
               20
<210> 159
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by NHNHAc
<400> 159
   Phe Cys Thr Gly Ile Arg Val Ala His Leu Ala Leu Lys His Arg Gln
   1               5                   10                  15
   Gly Lys Asn His
               20
<210> 160
   <211> 20
   <212> PRT
   <213> Artificial Sequence
<220>
   <223> N-acyleicosapeptide derivative
<220>
   <221> MOD_RES
   <222> (1)..(1)
   <223> modified by Ac
<220>
   <221> MOD_RES
   <222> (20)..(20)
   <223> modified by NHOH
<400> 160
   Phe Cys Thr Gly Ile Arg Val Ala His Leu Ala Leu Lys His Arg Gln
   1               5                   10                  15
   Gly Lys Asn His
               20
<210> 161
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by OH
<400> 161
   Phe Cys Thr Gly Ile Arg Val Ala His Leu Ala Leu Lys His Arg Gln
   1               5                   10                  15
   Gly Lys Asn His
   20
<210> 162
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by OEt
<400> 162
   Phe Cys Thr Gly Ile Arg Val Ala His Leu Ala Leu Lys His Arg Gln
   1 5 10 15
   Gly Lys Asn His
   20
<210> 163
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by NH2
<400> 163
   Phe Cys Thr Gly Ile Arg Val Ala His Leu Ala Leu Lys His Arg Gln
   1 5 10 15
   Gly Lys Asn His
   20
<210> 164
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by NHPr
<400> 164
   Phe Cys Thr Gly Ile Arg Val Ala His Leu Ala Leu Lys His Arg Gln
   1               5                   10                  15
   Gly Lys Asn His
               20
<210> 165
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by NHNH2
<400> 165
   Phe Cys Thr Gly Ile Arg Val Ala His Leu Ala Leu Lys His Arg Gln
   1               5                   10                  15
   Gly Lys Asn His
               20
<210> 166
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by NHNHPr
<400> 166
   Phe Cys Thr Gly Ile Arg Val Ala His Leu Ala Leu Lys His Arg Gln
   1               5                   10                  15
   Gly Lys Asn His
               20
<210> 167
   <211> 20
   <212> PRT
   <213> Artificial Sequence
<220>
   <223> N-acyleicosapeptide derivative
<220>
   <221> MOD_RES
   <222> (1)..(1)
   <223> modified by Pr
<220>
   <221> MOD_RES
   <222> (20)..(20)
   <223> modified by NHOH
<400> 167
   Phe Cys Thr Gly Ile Arg Val Ala His Leu Ala Leu Lys His Arg Gln
   1               5                   10                  15
   Gly Lys Asn His
               20
<210> 168
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by OH
<400> 168
   Phe Cys Thr Gly Ile Arg Val Ala His Leu Ala Leu Lys His Arg Gln
   1               5                   10                  15
   Gly Lys Asn His
               20
<210> 169
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by OEt
<400> 169
   Phe Cys Thr Gly Ile Arg Val Ala His Leu Ala Leu Lys His Arg Gln
   1               5                   10                  15
   Gly Lys Asn His
               20
<210> 170
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by NH2
<400> 170
   Phe Cys Thr Gly Ile Arg Val Ala His Leu Ala Leu Lys His Arg Gln
   1               5                   10                  15
   Gly Lys Asn His
               20
<210> 171
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by NHBz
<400> 171
   Phe Cys Thr Gly Ile Arg Val Ala His Leu Ala Leu Lys His Arg Gln
   1               5                   10                  15
   Gly Lys Asn His
               20
<210> 172
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by NHNH2
<400> 172
   Phe Cys Thr Gly Ile Arg Val Ala His Leu Ala Leu Lys His Arg Gln
   1               5                   10                  15
   Gly Lys Asn His
               20
<210> 173
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by NHNHBz
<400> 173
   Phe Cys Thr Gly Ile Arg Val Ala His Leu Ala Leu Lys His Arg Gln
   1               5                   10                  15
   Gly Lys Asn His
               20
<210> 174
   <211> 20
   <212> PRT
   <213> Artificial Sequence
<220>
   <223> N-acyleicosapeptide derivative
<220>
   <221> MOD_RES
   <222> (1)..(1)
   <223> modified by Bz
<220>
   <221> MOD_RES
   <222> (20)..(20)
   <223> modified by NHOH
<400> 174
   Phe Cys Thr Gly Ile Arg Val Ala His Leu Ala Leu Lys His Arg Gln
   1               5                   10                  15
   Gly Lys Asn His
               20